# EUROPEAN PATENT APPLICATION

(11) **EP 3 919 506 A1**
(43) Date of publication of application: **08.12.2021**
(21) Application number: 20749538.3
(22) Date of filing: 30.01.2020
(51) Int. Cl.: C07K 14/435, C12N 1/21, C12N 15/12, C12N 15/67, C12N 15/90, C12P 21/02

(54) **METHOD FOR PRODUCING RECOMBINANT PROTEIN**

(30) Priority: 31.01.2019 JP 2019015716; 01.08.2019 JP 2019142388
(71) Applicant: Spiber Inc., Tsuruoka-shi, Yamagata 997-0052 (JP)
(72) Inventor: NAKAHIGASHI, Kenji, Tsuruoka-shi, Yamagata 997-0052 (JP); JIANG, Li, Tsuruoka-shi, Yamagata 997-0052 (JP); NODA, Takanobu, Tsuruoka-shi, Yamagata 997-0052 (JP); TSUYAMA, Kenji, Tsuruoka-shi, Yamagata 997-0052 (JP)
(74) Representative: Handley, Matthew Edward
(86) International application number: PCT/JP2020/003487
(87) International publication number: WO 2020/158877

(57) **Abstract**

The present invention relates to a production method for a recombinant protein, the production method including a growth reduction step of reducing cell growth of recombinant cells which express a recombinant protein and a production step of producing the recombinant protein by culturing the recombinant cells in a protein production culture medium in a state of reducing the cell growth, in which the cell growth of the recombinant cells is reduced in the growth reduction step by using recombinant cells having at least one modified morphogenetic regulator as the recombinant cells.

### [Sequence Listing]

SEQUENCE LISTING

<110>  Spiber Inc.

<120>  A method of producing recombinant protein

<130>  FP19-1358-00

<160>  11

<170>  PatentIn version 3.5

<210>  1
<211>  347
<212>  PRT
<213>  Escherichia coli

<400>  1

Met Leu Lys Lys Phe Arg Gly Met Phe Ser Asn Asp Leu Ser Ile Asp
1               5                   10                  15

Leu Gly Thr Ala Asn Thr Leu Ile Tyr Val Lys Gly Gln Gly Ile Val
            20                  25                  30

Leu Asn Glu Pro Ser Val Val Ala Ile Arg Gln Asp Arg Ala Gly Ser
        35                  40                  45

Pro Lys Ser Val Ala Ala Val Gly His Asp Ala Lys Gln Met Leu Gly
    50                  55                  60

Arg Thr Pro Gly Asn Ile Ala Ala Ile Arg Pro Met Lys Asp Gly Val
65                  70                  75                  80

Ile Ala Asp Phe Phe Val Thr Glu Lys Met Leu Gln His Phe Ile Lys
                85                  90                  95

Gln Val His Ser Asn Ser Phe Met Arg Pro Ser Pro Arg Val Leu Val
            100                 105                 110

Cys Val Pro Val Gly Ala Thr Gln Val Glu Arg Arg Ala Ile Arg Glu
        115                 120                 125

Ser Ala Gln Gly Ala Gly Ala Arg Glu Val Phe Leu Ile Glu Glu Pro
    130                 135                 140

Met Ala Ala Ala Ile Gly Ala Gly Leu Pro Val Ser Glu Ala Thr Gly
145                 150                 155                 160

Ser Met Val Val Asp Ile Gly Gly Gly Thr Thr Glu Val Ala Val Ile
                165                 170                 175

Ser Leu Asn Gly Val Val Tyr Ser Ser Ser Val Arg Ile Gly Gly Asp
                180                 185                 190

Arg Phe Asp Glu Ala Ile Ile Asn Tyr Val Arg Arg Asn Tyr Gly Ser
        195                 200                 205

Leu Ile Gly Glu Ala Thr Ala Glu Arg Ile Lys His Glu Ile Gly Ser
    210                 215                 220

Ala Tyr Pro Gly Asp Glu Val Arg Glu Ile Glu Val Arg Gly Arg Asn
225                 230                 235                 240

Leu Ala Glu Gly Val Pro Arg Gly Phe Thr Leu Asn Ser Asn Glu Ile
                245                 250                 255

Leu Glu Ala Leu Gln Glu Pro Leu Thr Gly Ile Val Ser Ala Val Met
                260                 265                 270

Val Ala Leu Glu Gln Cys Pro Pro Glu Leu Ala Ser Asp Ile Ser Glu
        275                 280                 285

Arg Gly Met Val Leu Thr Gly Gly Gly Ala Leu Leu Arg Asn Leu Asp
    290                 295                 300

Arg Leu Leu Met Glu Glu Thr Gly Ile Pro Val Val Val Ala Glu Asp
305                 310                 315                 320

Pro Leu Thr Cys Val Ala Arg Gly Gly Gly Lys Ala Leu Glu Met Ile
                325                 330                 335

Asp Met His Gly Gly Asp Leu Phe Ser Glu Glu
                340                 345

<210>  2
<211>  1179
<212>  PRT
<213>  Artificial Sequence

<220>
<223>  PRT966

<400>  2

Met Gly Pro Gly Val Phe Gly Pro Tyr Gly Pro Gly Ala Ser Ala Ala
1               5                   10                  15

Ala Ala Ala Gly Ile Asn Gly Pro Gly Ser Gly Val Phe Gly Pro Gly
                20                  25                  30

Ile Ser Gly Ile Tyr Gly Pro Gly Val Phe Gly Pro Gly Val Phe Gly

        35                  40                  45

Pro Gly Ser Ser Ala Ala Ala Ala Ala Gly Pro Gly Ile Tyr Gly Pro
    50                  55                  60

Gly Val Phe Gly Pro Ser Ala Ser Ala Ala Ala Ala Ala Gly Pro Gly
65                  70                  75                  80

Ser Gly Val Phe Gly Pro Gly Ala Ser Gly Ile Tyr Gly Pro Gly Val
                85                  90                  95

Phe Gly Pro Gly Val Phe Gly Pro Gly Ser Ser Ala Ala Ala Ala Ala
            100                 105                 110

Gly Ile Tyr Gly Ser Gly Pro Gly Val Phe Gly Pro Tyr Gly Ser Ala
        115                 120                 125

Ala Ala Ala Ala Gly Pro Gly Ser Gly Ile Tyr Gly Ile Gly Pro Tyr
    130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
                165                 170                 175

Gly Ile Tyr Gly Pro Tyr Ala Ser Ala Ala Ala Ala Ala Gly Ile Tyr
            180                 185                 190

Gly Ser Gly Pro Gly Val Phe Gly Pro Tyr Gly Pro Gly Ile Ser Gly
        195                 200                 205

Ser Gly Val Phe Gly Pro Gly Val Phe Gly Pro Tyr Ala Ser Ala Ala
    210                 215                 220

Ala Ala Ala Gly Pro Gly Val Phe Gly Pro Tyr Gly Pro Gly Ser Ser
225                 230                 235                 240

Ala Ala Ala Ala Ala Gly Ile Tyr Gly Tyr Gly Pro Gly Val Phe Gly
                245                 250                 255

Pro Tyr Gly Pro Gly Ala Ser Gly Ile Asn Gly Pro Gly Ser Gly Ile
            260                 265                 270

Tyr Gly Pro Gly Val Phe Gly Pro Gly Ile Ser Ala Ala Ala Ala Ala
        275                 280                 285

Gly Pro Gly Val Phe Gly Pro Tyr Gly Pro Gly Ala Ser Ala Ala Ala
    290                 295                 300

Ala Ala Gly Ile Tyr Gly Pro Gly Val Phe Gly Pro Gly Ile Tyr Gly
305                 310                 315                 320

Pro Gly Ser Ser Gly Pro Gly Val Phe Gly Pro Tyr Gly Pro Gly Ser
                325                 330                 335

Ser Ala Ala Ala Ala Ala Gly Ile Tyr Gly Pro Gly Val Phe Gly Pro
            340                 345                 350

Tyr Gly Pro Gly Ile Ser Ala Ala Ala Ala Ala Gly Ile Tyr Val Phe
        355                 360                 365

Gly Pro Gly Val Phe Gly Pro Tyr Gly Pro Gly Ala Ser Gly Pro Gly
    370                 375                 380

Val Phe Gly Pro Tyr Gly Pro Gly Ala Ser Ala Ala Ala Ala Ala Gly
385                 390                 395                 400

Pro Gly Ile Tyr Gly Pro Gly Val Phe Gly Pro Ser Ala Ser Ala Ala
                405                 410                 415

Ala Ala Ala Gly Ile Tyr Gly Ser Gly Pro Gly Ile Tyr Gly Pro Tyr
            420                 425                 430

Gly Pro Gly Ile Ser Gly Pro Gly Ser Gly Val Phe Gly Ile Gly Pro
        435                 440                 445

Tyr Gly Pro Gly Ala Ser Ala Ala Ala Ala Ala Gly Ile Tyr Gly Pro
    450                 455                 460

Gly Val Phe Gly Pro Tyr Gly Pro Gly Ile Ser Ala Ala Ala Ala Ala
465                 470                 475                 480

Gly Pro Gly Ser Gly Ile Tyr Gly Pro Gly Ala Ser Gly Ile Asn Gly
                485                 490                 495

Pro Gly Ser Gly Ile Tyr Gly Pro Gly Val Phe Gly Pro Gly Ile Ser
            500                 505                 510

Ala Ala Ala Ala Ala Gly Ile Tyr Val Phe Gly Pro Gly Val Phe Gly
        515                 520                 525

Pro Tyr Gly Pro Gly Ala Ser Ala Ala Ala Ala Ala Gly Ile Tyr Gly
    530                 535                 540

Ser Gly Pro Gly Val Phe Gly Pro Tyr Gly Pro Gly Ile Ser Gly Ser
545                 550                 555                 560

Gly Val Phe Gly Pro Gly Val Phe Gly Pro Tyr Ala Ser Ala Ala Ala
                565                 570                 575

Ala Ala Gly Pro Gly Ser Gly Val Phe Gly Pro Gly Ala Ser Gly Pro
            580                 585                 590

Gly Val Phe Gly Pro Tyr Gly Pro Gly Ala Ser Ala Ala Ala Ala Ala
        595                 600                 605

Gly Ile Asn Gly Pro Gly Ser Gly Val Phe Gly Pro Gly Ile Ser Gly
    610                 615                 620

Ile Tyr Gly Pro Gly Val Phe Gly Pro Gly Val Phe Gly Pro Gly Ser
625                 630                 635                 640

Ser Ala Ala Ala Ala Ala Gly Pro Gly Ile Tyr Gly Pro Gly Val Phe
                645                 650                 655

Gly Pro Ser Ala Ser Ala Ala Ala Ala Ala Gly Pro Gly Ser Gly Val
            660                 665                 670

Phe Gly Pro Gly Ala Ser Gly Ile Tyr Gly Pro Gly Val Phe Gly Pro
        675                 680                 685

Gly Val Phe Gly Pro Gly Ser Ser Ala Ala Ala Ala Ala Gly Ile Tyr
    690                 695                 700

Gly Ser Gly Pro Gly Val Phe Gly Pro Tyr Gly Ser Ala Ala Ala Ala
705                 710                 715                 720

Ala Gly Pro Gly Ser Gly Ile Tyr Gly Ile Gly Pro Tyr Gly Pro Gly
                725                 730                 735

Ala Ser Gly Pro Gly Ile Tyr Gly Pro Gly Val Phe Gly Pro Ser Ala
            740                 745                 750

Ser Ala Ala Ala Ala Ala Gly Ser Gly Val Phe Gly Pro Gly Ile Tyr
        755                 760                 765

Gly Pro Tyr Ala Ser Ala Ala Ala Ala Ala Gly Ile Tyr Gly Ser Gly
    770                 775                 780

Pro Gly Val Phe Gly Pro Tyr Gly Pro Gly Ile Ser Gly Ser Gly Val
785                 790                 795                 800

Phe Gly Pro Gly Val Phe Gly Pro Tyr Ala Ser Ala Ala Ala Ala Ala
                805                 810                 815

Gly Pro Gly Val Phe Gly Pro Tyr Gly Pro Gly Ser Ser Ala Ala Ala
            820                 825                 830

Ala Ala Gly Ile Tyr Gly Tyr Gly Pro Gly Val Phe Gly Pro Tyr Gly
        835                 840                 845

Pro Gly Ala Ser Gly Ile Asn Gly Pro Gly Ser Gly Ile Tyr Gly Pro
    850                 855                 860

Gly Val Phe Gly Pro Gly Ile Ser Ala Ala Ala Ala Ala Gly Pro Gly
865                 870                 875                 880

Val Phe Gly Pro Tyr Gly Pro Gly Ala Ser Ala Ala Ala Ala Ala Gly
                885                 890                 895

Ile Tyr Gly Pro Gly Val Phe Gly Pro Gly Ile Tyr Gly Pro Gly Ser
            900                 905                 910

Ser Gly Pro Gly Val Phe Gly Pro Tyr Gly Pro Gly Ser Ser Ala Ala
        915                 920                 925

Ala Ala Ala Gly Ile Tyr Gly Pro Gly Val Phe Gly Pro Tyr Gly Pro
    930                 935                 940

Gly Ile Ser Ala Ala Ala Ala Ala Gly Ile Tyr Val Phe Gly Pro Gly
945                 950                 955                 960

Val Phe Gly Pro Tyr Gly Pro Gly Ala Ser Gly Pro Gly Val Phe Gly
                965                 970                 975

Pro Tyr Gly Pro Gly Ala Ser Ala Ala Ala Ala Ala Gly Pro Gly Ile
            980                 985                 990

Tyr Gly Pro Gly Val Phe Gly Pro  Ser Ala Ser Ala Ala  Ala Ala Ala
        995                 1000                1005

Gly Ile  Tyr Gly Ser Gly Pro  Gly Ile Tyr Gly Pro  Tyr Gly Pro
    1010                1015                1020

Gly Ile  Ser Gly Pro Gly Ser  Gly Val Phe Gly Ile  Gly Pro Tyr
    1025                1030                1035

Gly Pro  Gly Ala Ser Ala Ala  Ala Ala Ala Gly Ile  Tyr Gly Pro

1040                 1045                 1050

Gly Val  Phe Gly Pro Tyr Gly  Pro Gly Ile Ser Ala  Ala Ala Ala
    1055                 1060                 1065

Ala Gly  Pro Gly Ser Gly Ile  Tyr Gly Pro Gly Ala  Ser Gly Ile
    1070                 1075                 1080

Asn Gly  Pro Gly Ser Gly Ile  Tyr Gly Pro Gly Val  Phe Gly Pro
    1085                 1090                 1095

Gly Ile  Ser Ala Ala Ala Ala  Ala Gly Ile Tyr Val  Phe Gly Pro
    1100                 1105                 1110

Gly Val  Phe Gly Pro Tyr Gly  Pro Gly Ala Ser Ala  Ala Ala Ala
    1115                 1120                 1125

Ala Gly  Ile Tyr Gly Ser Gly  Pro Gly Val Phe Gly  Pro Tyr Gly
    1130                 1135                 1140

Pro Gly  Ile Ser Gly Ser Gly  Val Phe Gly Pro Gly  Val Phe Gly
    1145                 1150                 1155

Pro Tyr  Ala Ser Ala Ala Ala  Ala Ala Gly Pro Gly  Ser Gly Val
    1160                 1165                 1170

Phe Gly  Pro Gly Ala Ser
    1175

<210>  3
<211>  12
<212>  PRT
<213>  Artificial Sequence

<220>
<223>  His tag

<400>  3

Met His His His His His His Ser Ser Gly Ser Ser
1               5                   10

<210>  4
<211>  22
<212>  DNA
<213>  Artificial Sequence

<220>
<223>  A2T-F primer

<400>  4
agcgtaactg cagtaggtca tg                                                22

<210>  5
<211>  22
<212>  DNA
<213>  Artificial Sequence

<220>
<223>  A2T-R primer

<400>  5
tactgcagtt acgcttttcg gt                                                22


<210>  6
<211>  169
<212>  PRT
<213>  Escherichia coli

<400>  6

Met Tyr Thr Ser Gly Tyr Ala His Arg Ser Ser Ser Phe Ser Ser Ala
1               5                   10                  15

Ala Ser Lys Ile Ala Arg Val Ser Thr Glu Asn Thr Thr Ala Glu Leu
            20                  25                  30

Ile Ser Glu Val Val Tyr Arg Glu Asp Gln Pro Met Met Thr Gln Leu
        35                  40                  45

Leu Leu Leu Pro Leu Leu Gln Gln Leu Gly Gln Gln Ser Arg Trp Gln
    50                  55                  60

Leu Trp Leu Thr Pro Gln Gln Lys Leu Ser Arg Glu Trp Val Gln Ala
65                  70                  75                  80

Ser Gly Leu Pro Leu Thr Lys Val Met Gln Ile Ser Gln Leu Ser Pro
                85                  90                  95

Cys His Thr Val Glu Ser Met Val Arg Ala Leu Arg Thr Gly Asn Tyr
            100                 105                 110

Ser Val Val Ile Gly Trp Leu Ala Asp Asp Leu Thr Glu Glu Glu His
        115                 120                 125

Ala Glu Leu Val Asp Ala Ala Asn Glu Gly Asn Ala Met Gly Phe Ile
    130                 135                 140

Met Arg Pro Val Ser Ala Ser Ser His Ala Thr Arg Gln Leu Ser Gly
145                 150                 155                 160

Leu Lys Ile His Ser Asn Leu Tyr His
                165

<210>  7
<211>  510
<212>  DNA
<213>  Escherichia coli

<400>  7
atgtacactt caggctatgc acatcgttct tcgtcgttct catccgcagc aagtaaaatt        60

gcgcgtgtct ctacggaaaa cactacagcc gagcttatca gtgaagttgt ctatcgcgaa       120

gatcagccca tgatgacgca acttctactg ttgccattgt tacagcaact cggtcagcaa       180

tcgcgctggc aactctggtt aacaccgcaa caaaaactga gtcgggaatg ggttcaggca       240

tctgggctac ccttaacgaa agtaatgcag attagccagc tctccccttg ccacactgtg       300

gagtcaatgg ttcgcgcttt acgcacgggc aattacagtg tggtgatcgg ttggttggca       360

gatgatttga ctgaagaaga gcatgctgaa cttgttgatg cggcaaatga aggtaacgct       420

atggggttta ttatgcgtcc ggtaagcgca tcctctcacg ccacgagaca actttccggg       480

ctaaaaattc actctaattt gtatcattaa                                        510


<210>  8
<211>  42
<212>  DNA
<213>  Artificial Sequence

<220>
<223>  sulA_F primer

<400>  8
tttaagaagg agatatacat atgtacactt caggctatgc ac                          42


<210>  9
<211>  55
<212>  DNA
<213>  Artificial Sequence

<220>
<223>  sulA_R primer

<400>  9
tgtcgacgga gctcgaattc ttaatgatac aaattagagt gaatttttag cccgg            55


<210>  10
<211>  30
<212>  DNA
<213>  Artificial Sequence

<220>
<223>  RBS-4 primer

<400>  10
atgtatatct ccttcttaaa gttaaacaaa                                        30

<210>  11
<211>  19
<212>  DNA
<213>  Artificial Sequence

<220>
<223>  pET-MCS_F primer

<400>  11
gaattcgagc tccgtcgac                                                    19

## Description

### Technical Field

The present invention relates to a production method for a recombinant protein. The present invention also relates to a method for increasing a production volume of a recombinant protein per cell.

### Background Art

It is known that active cell growth is not necessarily linked to a recombinant protein producing ability in production of the recombinant protein using recombinant cells. For example, Patent Literature 1 discloses a method for producing a fibroin-like protein, the method including culturing *Escherichia coli* having a gene coding for a fibroin-like protein in a culture medium, inducing expression of the gene coding for the fibroin-like protein, and collecting the fibroin-like protein, in which cell growth after inducing the expression is reduced.

### Citation List

### Patent Literature

Patent Literature 1: WO 2015/178466 A

### Summary of Invention

### Technical Problem

An object of the present invention is to provide a production method for a recombinant protein which increases a recombinant protein producing ability while reducing cell growth of recombinant cells.

### Solution to Problem

The present inventors found that the cell growth of recombinant cells is reduced, and a production volume of a recombinant protein per cell is increased by using cells having a modified morphogenetic regulator as hosts in the production of the recombinant protein. The present invention is based on this novel finding.

For example, the present invention relates to each of the following inventions.
[1] A production method for a recombinant protein, the production method including a growth reduction step of reducing cell growth of recombinant cells which express a recombinant protein and a production step of producing the recombinant protein by culturing the recombinant cells in a protein production culture medium in a state of reducing the cell growth, in which the cell growth of the recombinant cells is reduced in the growth reduction step by using recombinant cells having at least one modified morphogenetic regulator as the recombinant cells.
[2] The production method according to [1], in which the modified morphogenetic regulator is a mutant cytoskeletal protein.
[3] The production method according to [2], in which the mutant cytoskeletal protein is mutant MreB.
[4] The production method according to [3], in which the mutant morphogenetic regulator has an amino acid sequence having at least 90% sequence identity with MreB.
[5] The production method according to [3] or [4], in which the mutant morphogenetic regulator has a mutation at the 53^{rd} amino acid residue of MreB, which is alanine.
[6] The production method according to any one of [3] to [5], in which the mutant morphogenetic regulator has a mutation that substitutes the 53^{rd} amino acid residue of MreB, which is alanine, for threonine.
[7] The production method according to [1], in which the recombinant cells are cells into which an expression cassette of a protein which controls a function of a cytoskeletal protein is introduced.
[8] The production method according to [7], in which the protein which controls the function of the cytoskeletal protein is sulA.
[9] The production method according to any one of [1] to [8], in which the protein production culture medium contains a naturally derived component.
[10] The production method according to any one of [1] to [9], in which a hydrophobicity of the recombinant protein is -1.0 or higher.
[11] The production method according to any one of [1] to [10], in which the recombinant protein is a structural protein.
[12] The production method according to any one of [1] to [11], in which the recombinant protein is fibroin.
[13] The production method according to any one of [1] to [12], in which the recombinant protein is spider silk fibroin.
[14] The production method according to any one of [1] to [13], in which the recombinant cells are bacilli.
[15] The production method according to any one of [1] to [14], in which the recombinant cells are microorganisms belonging to the genus *Escherichia.*
[16] A method for increasing a production volume of a recombinant protein per cell, the method including a growth reduction step of reducing cell growth of recombinant cells which express a recombinant protein and a production step of producing the recombinant protein by culturing the recombinant cells in a protein production culture medium in a state of reducing the cell growth, in which the cell growth of the recombinant cells is reduced in the growth reduction step by using recombinant cells having at least one modified morphogenetic regulator as the recombinant cells.
[17] The method according to [16], in which the modified morphogenetic regulator is a mutant cytoskeletal protein.
[18] The method according to [16], in which the recombinant cells are cells into which an expression cassette of a protein which controls a function of a cytoskeletal protein is introduced.

### Advantageous Effects of Invention

According to the present invention, a production method for a recombinant protein can be provided, which increases a recombinant protein producing ability while reducing cell growth of recombinant cells.

### Brief Description of Drawings

Fig. 1 is a graph obtained by plotting average particle sizes of a MreB mutant strain and a wild-type MreB strain against culturing time.
Fig. 2 is a graph showing weights of cells of the MreB mutant strain and cells of the wild-type MreB strain themselves (weights obtained by subtracting a weight of a produced recombinant protein from a dry cell weight) 16 hours after the start of production culturing.
Fig. 3 is a graph showing growth of the cells of the MreB mutant strain and the cells of the wild-type MreB strain 16 hours after the start of the production culturing.
Fig. 4 is a graph showing modified fibroin production volumes (production volumes per cell) in the MreB mutant strain and the wild-type MreB strain.
Fig. 5 is a graph showing modified fibroin production volumes (production volumes per culture medium) in the MreB mutant strain and the wild-type MreB strain.
Fig. 6 is a graph obtained by plotting average particle sizes of a sulA induction expression strain and a control strain against culturing time.
Fig. 7 is a graph obtained by plotting cell growth (cell concentrations) of the sulA induction expression strain and the control strain against culturing time.
Fig. 8 is a graph showing cell counts of the sulA induction expression strain and the control strain 24 hours after the start of production culturing.
Fig. 9 is a graph showing modified fibroin production volumes (production volumes per cell) in the sulA induction expression strain and the control strain.
Fig. 10 is a graph showing modified fibroin production volumes (production volumes per culture medium) in the sulA induction expression strain and the control strain.
Fig. 11 is a schematic diagram illustrating an outline of a method for integrating a modified fibroin expression cassette into host genomic DNA using a mechanism of lysogenization by HK022 phage.
Fig. 12 is a schematic diagram illustrating an outline of a method for integrating a modified fibroin expression cassette into host genomic DNA using a mechanism of lysogenization by ϕ80 phage.
Fig. 13 is a schematic diagram illustrating an outline of a method for integrating a modified fibroin expression cassette into host genomic DNA using a homologous recombination system of λ phage.

### Description of Embodiments

Hereinafter, embodiments of the present invention will be described in detail. However, the present invention is not limited to the following embodiments.

### [Production method for recombinant protein]

A production method for a recombinant protein according to the present embodiment includes at least a growth reduction step of reducing cell growth of recombinant cells which express a recombinant protein and a production step of producing the recombinant protein by culturing the recombinant cells in a protein production culture medium in a state of reducing the cell growth. Furthermore, the production method for a recombinant protein according to the present embodiment is a method in which the cell growth of the recombinant cells is reduced in the growth reduction step by using recombinant cells having at least one modified morphogenetic regulator as the recombinant cells.

### (Morphogenetic regulator)

As used herein, the "morphogenetic regulator" refers to a protein related to cell morphogenesis or control of cell morphology. Cell morphology includes, for example, the stiffness, shape, and size of a cell. Examples of the morphogenetic regulator can include a protein related to the formation or control of cell elongation, cell width, and cell polarity. Specific examples of the morphogenetic regulator can include a cytoskeletal protein, a protein that controls a function of a cytoskeletal protein, a peptidoglycan synthase, and the like.

In prokaryotic cells, for example, many bacteria such as *Escherichia coli,* the cells are covered with peptidoglycan (sugar chains cross-linked by short peptides). In these bacteria, degradation of already existing peptidoglycan and insertion of newly synthesized peptidoglycan are strictly controlled during elongation and division of cells, so that it is possible to maintain the form of the cells without rupture. One of the functions of the cytoskeletal protein is considered to be to control intracellular localization of a peptidoglycan synthase. That is, although the peptidoglycan in the periplasmic region finally determines the form of the cell, the cytoskeletal protein in the cytoplasm controls the enzyme that synthesizes the peptidoglycan.

Examples of the cytoskeletal protein of bacteria can include FtsZ tubulin and MreB actin. Examples of the peptidoglycan synthase can include PBP3 (FtsI) and PBP2. FtsZ tubulin is the first of division-related proteins to localize to the division plane and form a division ring (Z-ring). Furthermore, more than a dozen related proteins are assembled one after another at the Z-ring to form a supramolecular complex called divisome with PBP3. MreB forms a complex called elongasome with PBP2 and the like, which are essential for cell elongation. Examples of other components of the elongasome can include RodZ, RodA, MreC, MreD, and the like.

Examples of the cytoskeletal protein of eukaryotic cells can include crescentin, ParM and SopA of the cytoskeleton, and the like.

### (Modified morphogenetic regulator)

As used herein, the "modified morphogenetic regulator" refers to a morphogenetic regulator that has been modified by a substitution, deletion, insertion, addition, or mutation or artificial expression regulation. The artificial expression regulation means that expression of a nucleic acid or a gene is induced, decreased, or suppressed to induce, decrease, or suppress production of a protein or a polypeptide, respectively. An expression level of the morphogenetic regulator can be altered by integrating the morphogenetic regulator into an expression cassette and introducing the expression cassette into a cell. Furthermore, the expression level of the morphogenetic regulator can be altered by adding an enhancer or another regulatory sequence to the sequence of the morphogenetic regulator. Other modifications may also be included. Alternatively, a combination of the above may be used.

### (Mutant morphogenetic regulator)

As used herein, a "mutant morphogenetic regulator" refers to a morphogenetic regulator having an amino acid sequence corresponding to an amino acid sequence having a substitution, deletion, insertion, and/or addition of one or more amino acid residues as compared to the amino acid sequence of a wild-type morphogenetic regulator. Note that the mutant morphogenetic regulator includes a case in which the wild-type morphogenetic regulator is completely deleted (for example, the morphogenetic regulator is not expressed as a protein, since a gene coding for the morphogenetic regulator is missing from chromosomal DNA, the gene coding for the morphogenetic regulator is not expressed, or the like). The mutant morphogenetic regulator is preferably composed of an amino acid sequence having 90% or higher sequence identity with the amino acid sequence of the wild-type morphogenetic regulator, more preferably composed of an amino acid sequence having 95% or higher sequence identity with the amino acid sequence of the wild-type morphogenetic regulator, and even more preferably composed of an amino acid sequence having 99% or higher sequence identity with the amino acid sequence of the wild-type morphogenetic regulator. In the mutant morphogenetic regulator, a part or all of a biological activity of the wild-type morphogenetic regulator may be lost.

Cells having the mutant morphogenetic regulator can be obtained by, for example, a method of screening cells existing in nature, a method of performing screening after inducing a mutation, such as by treatment with a chemical such as A22 (S-(3,4-dichlorobenzyl)-isothiourea) and/or ultraviolet irradiation, and a method of obtaining cells having the mutant morphogenetic regulator by a genetic engineering technique.

Examples of the method using the genetic engineering technique can include a method of introducing a random mutation and a method of introducing a site-directed mutation. In the former method of introducing a random mutation, for example, a kit for introducing a random mutation (BD Diversify PCR Random Mutagenesis (manufactured by CLONTECH Laboratories, Inc.)) may be used. Furthermore, in the latter method of introducing a site-directed mutation, for example, a kit for introducing a site-directed mutation (Mutan-K (manufactured by Takara Bio Inc.)) may be used.

Among these methods, it is preferable to obtain the cells having the mutant morphogenetic regulator by the method using the genetic engineering technique, however, the present invention is not limited to this method.

The mutant morphogenetic regulator is preferably a mutant cytoskeletal protein and more preferably mutant MreB. The mutant MreB has an amino acid sequence corresponding to an amino acid sequence having a substitution, deletion, insertion, and/or addition of one or more amino acid residues as compared to the amino acid sequence of wild-type MreB (SEQ ID NO: 1).

Examples of the mutant MreB can include mutant MreB having a mutation at one or more amino acid residues such as the 14^{th} amino acid residue S, the 20^{th} amino acid residue A, the 23^{rd} amino acid residue L, the 53^{rd} amino acid residue A, the 74^{th} amino acid residue R, the 84^{th} amino acid residue F, the 143^{rd} amino acid residue E, the 158^{th} amino acid residue T, the 185^{th} amino acid residue S, the 207^{th} amino acid residue G, the 209^{th} amino acid residue L, the 276^{th} amino acid residue E, and the 322^{nd} amino acid residue L of the wild-type MreB (SEQ ID NO: 1). Preferred examples of the mutant MreB can include mutant MreB having a mutation at one or more amino acid residues selected from the 53^{rd} amino acid residue A, the 74^{th} amino acid residue R, the 84^{th} amino acid residue F, and the 185^{th} amino acid residue S of the wild-type MreB (SEQ ID NO: 1).

More specific examples of the mutant MreB can include mutant MreB having an amino acid sequence corresponding to an amino acid sequence having a substitution of one or more amino acid residues, for example, S14A, A20V, L23R, A53T, R74C, R74L, F84V, E143A, A158T, S185F, G207C, L209R, E276D, and L322Q, as compared to the wild-type MreB (SEQ ID NO: 1). The mutant MreB preferably has an amino acid sequence corresponding to an amino acid sequence having a substitution of one or more amino acid residues selected from E143A, R74L, A53T, S185F, and F84V and more preferably from R74L, A53T, S185F, F84V, G207C, and L208R as compared to the wild-type MreB (SEQ ID NO: 1), and more preferably has an amino acid sequence corresponding to an amino acid sequence having a substitution of one or more amino acid residues selected from A53T, S185F, and F84V as compared to the wild-type MreB (SEQ ID NO: 1).

### (Protein which controls function of cytoskeletal protein)

Examples of the protein which controls the function of the cytoskeletal protein, which is the morphogenetic regulator, in prokaryotes can include sulA, yeeV, slmA, and Min family proteins (MinC, D, and E). In addition, in some microorganisms such as the genus *Bacillus,* examples of the protein which controls the function of the cytoskeletal protein can include ezrA and Noc.

sulA is a component of the SOS system and inhibits polymerization of FtsZ by interacting with FtsZ. When sulA accumulates in a cell, the cell becomes a long filamentous cell without a septum (Journal of bacteriology, 1993, 175: 1118-1125). Wild-type sulA has the amino acid sequence listed as SEQ ID NO: 6, and a sulA gene coding for the wild-type sulA has, for example, the nucleic acid sequence listed as SEQ ID NO: 7. The nucleic acid sequence coding for the morphogenetic regulator sulA in this embodiment has at least 90%, preferably 93%, 95%, 98%, or 99% sequence identity with the nucleic acid sequence listed as SEQ ID NO: 7.

yeeV (CbtA) is a toxin of a type IV toxin-antitoxin (TA) system. yeeV acts in an inhibitory manner by interacting with each of FtsZ and MreB. In the case of FtsZ, yeeV inhibits GTP-dependent polymerization of FtsZ, and in the case of MreB, yeeV inhibits ATP-dependent polymerization of MreB. It is known that, since FtsZ and MreB control the size and form of a cell, the cell becomes large when yeeV which inhibits FtsZ and MreB is expressed excessively (Molecular microbiology, 2011, 79: 109-118 and PLoS genetics, 2017, 13: e1007007).

### (Recombinant cells having modified morphogenetic regulator)

The recombinant cells having the modified morphogenetic regulator include recombinant cells having a morphology regulator modified using artificial manipulation, such as a genetic engineering technique, of the wild-type morphogenetic regulator in the cells, recombinant cells having a morphogenetic regulator in nature which has undergone a mutation so that it is different from the wild-type morphogenetic regulator in the cells, recombinant cells in which the expression level of the morphogenetic regulator is altered by integrating the morphogenetic regulator into an expression cassette and introducing the expression cassette into the cells, and the like.

### (Recombinant cells)

The recombinant cells according to the present embodiment express the recombinant protein. The recombinant cells according to the present embodiment may be, for example, recombinant cells having a nucleic acid sequence coding for the recombinant protein (hereunder also referred to as "target protein") and one or more regulatory sequences linked in a functional manner to the nucleic acid sequence (hereunder may also be referred to as "target protein expression cassette"). The recombinant cells according to the present embodiment may have one expression cassette or multiple (for example, two, three, four, or five) expression cassettes.

The regulatory sequence is a sequence that controls an expression of the recombinant protein (target protein) in a host (for example, a promoter, an enhancer, a ribosome binding site, a transcription termination sequence, and the like), and can be selected as appropriate depending on the type of the host. The regulatory sequence may be exogenous or endogenous (host-derived regulatory sequence).

The recombinant cells having the target protein expression cassette can be obtained by, for example, a method of transforming the host cells with an expression vector containing at least the nucleic acid sequence coding for the target protein. The expression vector may contain the target protein expression cassette. The recombinant cells according to the present embodiment may have the target protein expression cassette outside the genomic DNA, or the target protein expression cassette may be integrated into the genomic DNA. It is preferable that the target protein expression cassette is integrated into the genomic DNA.

A known method can be used as a method for transforming the host cells, and examples thereof can include transforming the host cells using a plasmid vector.

A known method can be used as the method for integrating the target protein expression cassette into the genomic DNA, and examples thereof can include a λ red method to which a recombination mechanism in double-strand-break repair in λ phage is applied, a Red/ET homologous recombination method, and a transfer method utilizing a transposon activity in which pUT-mini Tn5 is used. For example, the target protein expression cassette can be integrated into the genomic DNA of the host cells using the "kit for introducing gene by transposon: pUTmini-Tn5 Kit" of Biomedal, S.L. according to the method described in the kit. In this case, the target protein expression cassette may be integrated into the genomic DNA of the host cells by causing recombination so that a DNA fragment containing at least the nucleic acid sequence coding for the target protein is linked in a functional manner to one or more regulatory sequences in the genomic DNA of the host cell.

As the method for transforming the host cells, a method of integrating the target protein expression cassette into the genomic DNA of the host cells via an attachment site in the genomic DNA of the host cell (attB site) and an attachment site in the vector (attP site) by integrase of λ phage and a method of integrating the target protein expression cassette into the genomic DNA of the host cells using a red recombinase system in which a helper plasmid pKD46 having three genes integral in homologous recombination, exo, bet, and gam, is used are preferred.

As the host cells, any of cells of a prokaryote such as bacteria and cells of a eukaryote such as yeast cells, filamentous fungal cells, insect cells, animal cells, and plant cells can be used. However, the host cells are preferably the cells of a prokaryote such as bacteria from the viewpoints of rapid growth and reduction in culturing cost. The host cells may be any of cocci, spirilla, and bacilli, and bacilli are preferred.

Examples of the prokaryotic host cells such as bacteria can include microorganisms belonging to the genera *Escherichia, Brevibacillus, Serratia, Bacillus, Microbacterium, Brevibacterium, Corynebacterium, Pseudomonas,* and the like. Preferable examples of the prokaryote can include *Escherichia coli, Bacillus subtilis, Pseudomonas, Corynebacterium, Lactococcus,* and the like. The host cells are preferably microorganisms belonging to the genus *Escherichia,* particularly *Escherichia coli.*

Examples of the microorganisms belonging to the genus *Escherichia* can include *Escherichia coli* BL21 (Novagen, Inc.), *Escherichia coli* BL21(DE3) (Life Technologies Corporation), *Escherichia coli* BLR(DE3) (Merck Millipore), *Escherichia coli* DH1, *Escherichia coli* GI698, *Escherichia coli* HB101, *Escherichia coli* JM109, *Escherichia coli* K5 (ATCC 23506), *Escherichia coli* KY3276, *Escherichia coli* MC1000, *Escherichia coli* MG1655 (ATCC 47076), *Escherichia coli* No.49, *Escherichia coli* Rosetta(DE3) (Novagen, Inc.), *Escherichia coli* TB1, *Escherichia coli* Tuner (Novagen, Inc.), *Escherichia coli* Tuner (DE3) (Novagen, Inc.), *Escherichia coli* W1485, *Escherichia coli* W3110 (ATCC 27325), *Escherichia coli* XL1-Blue, *Escherichia coli* XL2-Blue, and the like. It is preferable that the host cells are *Escherichia coli.*

As the method for transforming the host cells, any method can be used so long as it is a method for introducing DNA into the host cells. Examples of the method can include a method of using calcium ions [Proc. Natl. Acad. Sci. USA, 69, 2110 (1972)], a protoplast method (JP S63-248394 A), the method described in Gene, 17, 107 (1982) or Molecular & General Genetics, 168, 111 (1979), or the like.

Transformation of microorganisms belonging to the genus *Brevibacillus* can be carried out by, for example, a method of Takahashi et al. (J. Bacteriol., 1983, 156: 1130-1134), a method of Takagi et al. (Agric. Biol. Chem., 1989, 53: 3099-3100), or a method of Okamoto et al. (Biosci. Biotechnol. Biochem., 1997, 61: 202-203).

The type of a vector used for the transformation (hereunder simply referred to as "vector") can be selected as appropriate depending on the type of the host, such as a plasmid vector, a virus vector, a cosmid vector, a fosmid vector, an artificial chromosome vector, and the like. Examples of the vector can include pBTrp2, pBTac1, and pBTac2 (all commercially available from Boehringer Ingelheim GmbH), pKK233-2 (manufactured by Pharmacia), pSE280 (manufactured by Invitrogen Corporation), pGEMEX-1 (manufactured by Promega Corporation), pQE-8 (manufactured by QIAGEN), pKYP10 (JP S58-110600 A), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (manufactured by Stratagene), pTrs30 [prepared from *Escherichia coli* JM109/pTrS30 (FERM BP-5407)], pTrs32 [prepared from *Escherichia coli* JM109/pTrS32 (FERM BP-5408)], pGHA2 [prepared from *Escherichia coli* IGHA2 (FERM B-400), JP S60-221091 A], pGKA2 [prepared from *Escherichia coli* IGKA2 (FERM BP-6798), JP S60-221091 A], pTerm2 (US 4,686,191, US 4,939,094, and US 5,160,735), pSupex, pUB110, pTP5, pC194, pEG400 [J. Bacteriol., 172, 2392 (1990)], pGEX (manufactured by Pharmacia), pET System (manufactured by Novagen Inc.), and the like.

In a case where *Escherichia coli* is used as the host cells, examples of suitable vectors can include pUC18, pBluescript II, pSupex, pET22b, pCold, and the like.

Specific examples of a suitable vector for a microorganism belonging to the genus *Brevibacillus* can include pUB110 or pHY500 known as a *Bacillus subtilis* vector (JP H2-31682 A), pNY700 (JP H4-278091 A), pHY4831 (J. Bacteriol., 1987, 1239-1245), pNU200 (Shigezo Udaka, Journal of the Agricultural Chemical Society of Japan, 1987, 61: 669-676), pNU100 (Appl. Microbiol. Biotechnol., 1989, 30: 75-80), pNU211 (J. Biochem., 1992, 112: 488-491), pNU211R2L5 (JP H7-170984 A), pNH301 (Appl. Environ. Microbiol., 1992, 58: 525-531), pNH326, pNH400 (J. Bacteriol., 1995, 177: 745-749), pHT210 (JP H6-133782 A), pHT110R2L5 (Appl. Microbiol. Biotechnol., 1994, 42: 358-363), pNCO2 which is a shuttle vector between *Escherichia coli* and the microorganism belonging to the genus *Brevibacillus* (JP 2002-238569 A), and the like.

The promoter is not limited so long as it functions in the host cells. Examples of the promoter can include a promoter derived from *Escherichia coli,* a phage, or the like such as a trp promoter (Ptrp), a lac promoter, a PL promoter, a PR promoter, and a T7 promoter. It is also possible to use an artificially designed and modified promoter such as a promoter in which two Ptrp's are arranged in tandem (Ptrp×2), a tac promoter, a lacT7 promoter, and a let I promoter.

It is preferable to use a plasmid in which the distance between the Shine-Dalgarno sequence, which is a ribosome binding site, and the start codon is adjusted to an appropriate distance (for example, 6 to 18 bases). The transcription termination sequence is not necessarily required, however, it is preferable to arrange the transcription termination sequence immediately downstream of the gene coding for the target protein.

Examples of the eukaryotic host cells can include yeast and a filamentous fungus (mold or the like).

Examples of the yeast can include yeasts belonging to the genera Saccharomyces, Schizosaccharomyces, Kluyveromyces, Trichosporon, Schwanniomyces, Pichia, Candida, Yarrowia, Hansenula, and the like.

When yeast is used as the host cells, it is generally preferable that the vector contains an origin of replication (in a case where multiplication in the host cells is required), a selection marker for multiplication of the vector in *Escherichia coli,* an inducible promoter and a terminator for the expression of the recombinant protein in yeast, and a selection marker for yeast.

In a case where the vector is a nonintegrated vector, it is preferable that the vector further contains an autonomously replicating sequence (ARS). This allows the stability of the vector in the cells to be improved (Myers, A. M., et al. (1986) Gene 45: 299-310).

Examples of the vector when using yeast as the host cells can include YEP13 (ATCC 37115), YEp24 (ATCC 37051), YCp50 (ATCC 37419), YIp, pHS19, pHS15, pA0804, pHIL3Ol, pHIL-Sl, pPIC9K, pPICZα, pGAPZα, pPICZ B, and the like.

Specific examples of the promoter when yeast is used as the host cells can include galactose-inducible gall promoter and gal10 promoter; a copper-inducible CUP1 promoter; a thiamine-inducible nmtl promoter; and methanol-inducible AOX1 promoter, AOX2 promoter, DHAS promoter, DAS promoter, FDH promoter, FMDH promoter, MOX promoter, ZZA1, PEX5-, PEX8-, and PEX14-promoters, and the like.

Any method can be used as a method for introducing the vector into yeast, so long as it is a method for introducing DNA into yeast, and examples thereof can include an electroporation method (Methods Enzymol., 194, 182 (1990)), a spheroplast method (Proc. Natl. Acad. Sci., USA, 81, 4889 (1984)), a lithium acetate method (J. Bacteriol., 153, 163 (1983)), the method described in Proc. Natl. Acad. Sci. USA, 75, 1929 (1978), and the like.

Examples of the filamentous fungus can include fungi belonging to the genera Acremonium, Aspergillus, Ustilago, Trichoderma, Neurospora, Fusarium, Humicola, Penicillium, Myceliophtora, Botryts, Magnaporthe, Mucor, Metarhizium, Monascus, Rhizopus, and Rhizomucor, and the like.

Specific examples of the promoter when the filamentous fungus is used as the host cells can include a salicylic acid-inducible PR1a promoter; a cycloheximide-inducible Placc promoter; a quinic acid-inducible Pqa-2 promoter, and the like.

Introduction of the vector into the filamentous fungus can be carried out using a method known in the prior art. Examples of the method can include a method of Cohen et al. (calcium chloride method) [Proc. Natl. Acad. Sci. USA, 69: 2110 (1972)], a protoplast method [Mol. Gen. Genet., 168: 111 (1979)], a competent method [J. Mol. Biol., 56: 209 (1971)], an electroporation method, and the like.

In the preparation of the recombinant cells according to the present embodiment, the order of the integration of the target protein expression cassette and the introduction of a mutation into the morphogenetic regulator is not limited. That is, the target protein expression cassette may be integrated into the host cells having the modified morphogenetic regulator, or the modified morphogenetic regulator may be introduced into the host cells having the target protein expression cassette.

### (Target protein)

The target protein produced by the production method for a recombinant protein according to the present embodiment is not particularly limited, and any protein can be used. Here, the target protein refers to a protein intended to be produced by the production method according to the present embodiment and then recovered to be used. Examples of the target protein can include any protein which is preferably produced on an industrial scale, and examples thereof can include a protein which can be used for industrial use, a protein which can be used for medical use, a structural protein, and the like. Specific examples of the protein that can be used for industrial use or medical use can include an enzyme, a regulatory protein, a receptor, a peptide hormone, a cytokine, a membrane or transport protein, an antigen used for vaccination, a vaccine, an antigen-binding protein, an immunostimulatory protein, an allergen, a full-length antibody, an antibody fragment or derivative, and the like. Specific examples of the structural protein can include fibroin (For example, spider silk, silkworm silk, and the like), keratin, collagen, elastin, resilin, a fragment of these proteins, a protein derived from these proteins, and the like.

As used herein, the fibroin includes naturally derived fibroin and modified fibroin. As used herein, the "naturally derived fibroin" refers to fibroin having an amino acid sequence identical to that of naturally derived fibroin, and the "modified fibroin" refers to fibroin having an amino acid sequence different from that of the naturally derived fibroin.

The fibroin may be spider silk fibroin. The "spider silk fibroin" includes natural spider silk fibroin and modified fibroin derived from the natural spider silk fibroin. Examples of the natural spider silk fibroin can include spider silk proteins produced by arachnids.

The fibroin may be, for example, a protein having a domain sequence represented by Formula 1: [(A)ₙ motif-REP]ₘ or Formula 2: [(A)ₙ motif-REP]ₘ-(A)ₙ motif. An amino acid sequence (N-terminal sequence or C-terminal sequence) may be further added to any one or both of the N-terminal side and the C-terminal side of the domain sequence of the fibroin according to the present embodiment. The N-terminal sequence and the C-terminal sequence are typically regions not containing repeats of amino acid motifs that are characteristic of fibroin and are composed of about 100 residues of amino acids, but are not limited thereto.

As used herein, the "domain sequence" is an amino acid sequence giving rise to a crystalline region characteristic of fibroin (typically corresponds to the (A)ₙ motif in the amino acid sequence) and a non-crystalline region characteristic of fibroin (typically corresponds to REP in the amino acid sequence) and refers to an amino acid sequence represented by Formula 1: [(A)ₙ motif-REP]ₘ or Formula 2: [(A)ₙ motif-REP]ₘ-(A)ₙ motif. Here, the (A)ₙ motif represents an amino acid sequence mainly composed of alanine residues, and the number of amino acid residues therein is 2 to 27. The number of the amino acid residues in the (A)ₙ motif may be an integer of 2 to 20, 4 to 27, 4 to 20, 8 to 20, 10 to 20, 4 to 16, 8 to 16, or 10 to 16. In addition, a ratio of the number of alanine residues to the total number of the amino acid residues in the (A)ₙ motif may be 40% or higher, or may also be 60% or higher, 70% or higher, 80% or higher, 83% or higher, 85% or higher, 86% or higher, 90% or higher, 95% or higher, or 100% (meaning that the (A)ₙ motif is only composed of alanine residues). Among the multiple (A)ₙ motifs in the domain sequence, at least 7 (A)ₙ motifs may be only composed of alanine residues. REP represents an amino acid sequence composed of 2 to 200 amino acid residues. REP may also be an amino acid sequence composed of 10 to 200 amino acid residues. m represents an integer of 2 to 300, and may be an integer of 10 to 300. The multiple (A)ₙ motifs may be identical amino acid sequences or different amino acid sequences. The multiple REP sequences may be identical amino acid sequences or different amino acid sequences.

Examples of the naturally derived fibroin can include a protein having a domain sequence represented by Formula 1: [(A)ₙ motif-REP]ₘ or Formula 2: [(A)ₙ motif-REP]ₘ-(A)ₙ motif. Specific examples of the naturally derived fibroin can include fibroin produced by insects or arachnids.

Examples of the fibroin produced by insects can include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama and a hornet silk protein secreted by larvae of Vespa simillima xanthoptera.

More specific examples of the fibroin produced by insects can include the silkworm fibroin L chain (GenBank Accession Nos. M76430 (nucleotide sequence) and AAA27840.1 (amino acid sequence)).

Examples of the fibroin produced by arachnids can include spider silk proteins produced by spiders belonging to the genus *Araneus,* such as *Araneus ventricosus, Araneus diadematus, Araneus pinguis, Araneus pentagrammicus,* and *Araneus nojimai,* spiders belonging to the genus *Neoscona,* such as *Neoscona scylla, Neoscona nautica, Neoscona adianta,* and *Neoscona scylloides,* spiders belonging to the genus *Pronus,* such as *Pronous minutus,* spiders belonging to the genus *Cyrtarachne,* such as *Cyrtarachne bufo* and *Cyrtarachne inaequalis,* spiders belonging to the genus *Gasteracantha,* such as *Gasteracantha kuhlii* and *Gasteracantha mammosa,* spiders belonging to the genus *Ordgarius,* such as *Ordgarius hobsoni* and *Ordgarius sexspinosus,* spiders belonging to the genus *Argiope,* such as *Argiope amoena, Argiope minuta,* and *Argiope bruennichi,* spiders belonging to the genus *Arachnura,* such as *Arachnura logio,* spiders belonging to the genus *Acusilas,* such as Acusilas coccineus, spiders belonging to the genus *Cytophora,* such as *Cyrtophora moluccensis, Cyrtophora exanthematica,* and *Cyrtophora unicolor,* spiders belonging to the genus *Poltys,* such as *Poltys illepidus,* spiders belonging to the genus *Cyclosa,* such as *Cyclosa octotuberculata, Cyclosa sedeculata, Cyclosa vallata,* and *Cyclosa atrata,* and spiders belonging to the genus *Chorizopes,* such as *Chorizopes nipponicus,* and spider silk proteins produced by spiders belonging to the family *Tetragnathidae,* such as spiders belonging to the genus *Tetragnatha,* such as *Tetragnatha praedonia, Tetragnatha maxillosa, Tetragnatha extensa,* and *Tetragnatha squamata,* spiders belonging to the genus *Leucauge,* such as *Leucauge magnifica, Leucauge blanda,* and *Leucauge subblanda,* spiders belonging to the genus *Nephila,* such as *Nephila clavata* and *Nephila pilipes,* spiders belonging to the genus *Menosira,* such as *Menosira ornata,* spiders belonging to the genus *Dyschiriognatha,* such as *Dyschiriognatha tenera,* spiders belonging to the genus *Latrodectus,* such as *Latrodectus mactans, Latrodectus hasseltii, Latrodectus geometricus,* and *Latrodectus tredecimguttatus,* and spiders belonging to the genus *Euprosthenops.* Examples of the spider silk proteins can include dragline silk proteins such as MaSps (MaSpl and MaSp2) and ADFs (ADF3 and ADF4), MiSps (MiSp1 and MiSp2), and the like.

Examples of the keratin-derived protein can include a type I keratin of *Capra hircus,* and the like.

Examples of the collagen-derived protein can include a protein having a domain sequence represented by Formula 3: [REP2]ₚ (here, in Formula 3, p represents an integer of 5 to 300; REP2 represents an amino acid sequence composed of Gly-X-Y, and X and Y represents any amino acid residue other than Gly; and the multiple REP2 sequences may be identical amino acid sequences or different amino acid sequences).

Examples of the elastin-derived protein can include proteins having the amino acid sequences of NCBI GenBank Accession Nos. AAC98395 (human), 147076 (sheep), NP786966 (cow), and the like.

Examples of the resilin-derived protein can include a protein having a domain sequence represented by Formula 4: [REP3]_{q} (here, in Formula 4, q represents an integer of 4 to 300; REP3 represents an amino acid sequence composed of Ser-J-J-Tyr-Gly-U-Pro; J represents any amino acid residue, and an amino acid residue selected from the group consisting of Asp, Ser, and Thr is particularly preferred; U represents any amino acid residue, and an amino acid residue selected from the group consisting of Pro, Ala, Thr, and Ser is particularly preferred; and the multiple REP4 sequences may be identical amino acid sequences or different amino acid sequences).

The target protein may be a hydrophilic protein or a hydrophobic protein. For the target protein, a value obtained by adding up the hydropathy indices (HI) of all amino acid residues constituting the target protein and then dividing the sum by the total number of amino acid residues (average HI; hereunder also referred to as "hydrophobicity") is preferably -1.0 or greater. As the hydropathy index of an amino acid residue, a known index (Hydropathy index: Kyte J and Doolittle R (1982) "A simple method for displaying the hydropathic character of a protein", J. Mol. Biol., 157, pp. 105-132) is used. Specifically, the hydropathy index of each amino acid is indicated in the following Table 1.

**[Table 1]**

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

In an embodiment of the present invention, the hydrophobicity of the target protein may be -0.9 or higher, -0.8 or higher, -0.7 or higher, -0.6 or higher, -0.5 or higher, -0.4 or higher, -0.3 or higher, -0.2 or higher, - 0.1 or higher, 0 or higher, 0.1 or higher, 0.2 or higher, 0.3 or higher, or 0.4 or higher, and the hydrophobicity of the target protein may be 1.0 or lower, 0.9 or lower, 0.8 or lower, 0.7 or lower, 0.6 or lower, or 0.5 or lower.

A molecular weight of the target protein is not particularly limited, and may be, for example, 10 kDa or more and 700 kDa or less. The molecular weight of the target protein may be, for example, 20 kDa or more, 30 kDa or more, 40 kDa or more, 50 kDa or more, 60 kDa or more, 70 kDa or more, 80 kDa or more, 90 kDa or more, or 100 kDa or more, and may be, for example, 600 kDa or less, 500 kDa or less, 400 kDa or less, 300 kDa or less, or 200 kDa or less. In general, the greater the molecular weight of the protein is, the easier the aggregation of the protein tends to be.

### (Growth reduction step)

The growth reduction step is a step of reducing the cell growth of the recombinant cells which express the recombinant protein. In the production method for a recombinant protein according to the present embodiment, the cell growth of the recombinant cells is reduced by using the recombinant cells described above (the recombinant cells having at least one modified morphogenetic regulator) as the recombinant cells.

In the growth reduction step, the cell growth of the recombinant cells can be reduced by culturing the recombinant cells having at least one modified morphogenetic regulator in the protein production culture medium described later.

### (Production step)

The production step is a step of producing the recombinant protein by culturing the recombinant cells in a protein production culture medium in a state of reducing the cell growth. The growth reduction step and the production step can be carried out simultaneously.

The protein production culture medium for culturing the recombinant cells is not particularly limited, and can be selected from known natural culture medium or synthetic culture medium depending on the type of the recombinant cells. As the protein production culture medium, for example, a liquid culture medium containing components selected from a carbon source, a nitrogen source, a phosphoric acid source, a sulfur source, vitamins, a mineral, a nutrient required by auxotrophy, and other various organic components and inorganic components as necessary can be used. The type and concentration of the medium component may be set as appropriate by those skilled in the art.

It is preferable that the protein production culture medium contains a naturally derived component. The naturally derived component refers to a component such as a natural product (for example, yeast) itself or an extract of the natural product (for example, yeast extract). The type and content of each component contained in the naturally derived component are generally not completely specified. The naturally derived component contains, for example, at least one selected from the group consisting of vitamins, a low molecular weight peptide (for example, a peptide composed of 2 to 20 amino acid residues), and an amino acid.

Examples of the carbon source can include saccharides such as glucose, sucrose, lactose, galactose, fructose, and a hydrolyzate of starch, alcohols such as glycerol and sorbitol, and organic acids such as fumaric acid, citric acid, and succinic acid.

One kind of the carbon source may be contained in the culture medium, or a mixture of two or more kinds of the carbon sources may be contained at an arbitrary ratio. A concentration of the carbon source in the protein production culture medium may be about 0.1 w/v% to 50 w/v%, preferably about 0.5 w/v% to 40 w/v%, more preferably about 1 w/v% to 30 w/v%, and particularly preferably about 5 w/v% to 20 w/v%. In the present embodiment, it is preferable to use glycerol or glucose as the carbon source, and glycerol or glucose may be mixed with another carbon source at an arbitrary ratio. The ratio of glycerol or glucose in the carbon source is preferably 10 wt% or higher, more preferably 50 wt% or higher, and particularly preferably 70 wt% or higher. The preferred initial concentration of the carbon source at the start of the culturing is as described above, however, the carbon source may be added as appropriate depending on consumption of the carbon source during the culturing.

Examples of the nitrogen source can include an inorganic nitrogen salt such as a nitrate, an ammonium salt, an ammonia gas, and ammonia water and an organic nitrogen source such as an amino acid, peptones, extracts, corn steep liquor (CSL) which is a by-product in the corn starch production industry. Examples of the peptones can include casein peptone, meat peptone, heart muscle peptone, gelatin peptone, soy peptone, and the like. Examples of the extracts can include meat extract, yeast extract, heart infusion, and the like. In the nitrogen source containing an amino acid or a peptide, it is preferable that contents of the lower molecular weight peptide and the amino acid are high.

Examples of the phosphoric acid source can include a phosphate such as potassium dihydrogen phosphate and dipotassium hydrogen phosphate and a phosphoric acid polymer such as pyrophosphoric acid.

Examples of the sulfur source can include an inorganic sulfur compound such as a sulfate, a thiosulfate, and a sulfite and a sulfur-containing amino acid such as cysteine, cystine, and glutathione.

Examples of the vitamins can include biotin, choline chloride, cyanocobalamin, folic acid, inositol, nicotinic acid, 4-aminobenzoic acid, pantothenic acid, pyridoxine, riboflavin, thianmine, thymdine, and the like. Examples of sources of the vitamins can include various extracts such as malt extract, potato extract, and tomato juice.

Examples of the minerals can include sulfur (S), potassium (K), calcium (Ca), magnesium (Mg), iron (Fe), sodium (Na), and the like, in addition to phosphorus (P).

The culturing in the production step can be carried out aerobically by aeration culturing or shaking culturing. The culturing can be carried out by batch culture, fed-batch culture, continuous culture, or a combination thereof. A pH of the protein production culture medium may be, for example, 3.0 to 9.0. A culturing temperature may be, for example, 15 to 40°C. Culturing time may be, for example, 1 to 60 hours.

Culturing conditions are not particularly limited so long as the recombinant cells can grow therein, and the target protein can be accumulated in the recombinant cells which express the target protein. Note that, during the period of the target protein expression, the recombinant cells may or may not grow. The culturing conditions during the period before the target protein is expressed and the period after the expression of the target protein starts may or may not be the same as each other.

The culturing temperature generally has a significant effect on cell growth. Generally speaking, the lower limit temperature for growth is 0°C, which is the freezing temperature of moisture in the cell, or slightly lower than 0°C, and the upper limit temperature is determined by the denaturation temperature of a macromolecular compound such as a protein and a nucleic acid. A temperature range in which a certain strain can grow is relatively narrow. For example, in *Escherichia coli,* the lower limit temperature for growth is 0 to 15°C, the upper limit is 46°C, and the optimum temperature for growth is about 36 to 42°C. In a case where microorganisms are classified according to the optimum temperature for growth, the microorganisms are classified into psychrophilic bacteria having an optimum temperature of 20°C or lower, mesophilic bacteria having an optimum temperature of 20 to 45°C, and thermophilic bacteria having an optimum temperature of 45°C or higher. Here, the optimum temperature for growth refers to a temperature at which the microorganism to be cultured is at the maximum specific growth rate, and the specific growth rate refers to a growth rate per unit microorganism amount, is a value unique to a microorganism, and changes depending on the culture conditions.

In an embodiment of the present invention, the "optimum temperature for growth" refers to a temperature at which a microorganism can be at the maximum specific growth rate in a case where conditions other than the culturing temperature, such as a pH and a dissolved oxygen concentration, are constant at the start of culturing. In an embodiment of the present invention, when the recombinant cells express the target protein (after induction of the expression in a case where the expression of the target protein is inducible), the expression level of the target protein in the recombinant cells can be increased by cooling or maintaining the recombinant cells at a temperature lower than the optimum temperature for growth of the recombinant cells by adjustment of the culturing temperature or the like. The temperature lower than the optimum temperature for growth of the recombinant cells may be, for example, a temperature which is 3 to 25°C lower than the lower limit of the optimum temperature for growth of the recombinant cells, may be a temperature which is 8 to 20°C lower than the lower limit, may be a temperature which is 10 to 18°C lower than the lower limit, may be a temperature which is 12°C to 18°C lower than the lower limit, may be a temperature which is 14°C to 17°C lower than the lower limit, may be a temperature which is 3 to 10°C lower than the lower limit, or may be a temperature which is 5 to 8°C lower than the lower limit.

### (Induction of recombinant protein expression)

The recombinant cells of the present embodiment may be cells in which the expression of the target protein can be induced. The induction of the recombinant protein expression is carried out by activating transcription by an inducible promoter (transcription of a nucleic acid coding for a protein of interest). The activation of the inducible promoter can be carried out according to a method known in the technical field, depending on the type of inducible promoter.

For example, when an inducible promoter activated by the presence of an inducing substance (expression inducing agent) such as isopropyl-β-thiogalactopyranoside (IPTG) is used, the expression of the recombinant protein can be induced by adding the inducing substance to the culture solution. The inducing substance may be added to the culture solution all at once or in several portions, or it may be added to the culture solution as a continuous feed. Feeding may also be performed by addition of the inducing substance to the feed substrate solution. The amount of the inducing substance added can be set according to the type of the inducing substance and the inducible promoter, and for example, the amount can be in the range of 0.1 to 30 µg and preferably in the range of 0.5 to 20 µg, for 1 g of a dry weight of the recombinant cells.

When an inducible promoter activated by increase or decrease in the temperature is used, for example, expression of the recombinant protein can be induced by increasing or decreasing the temperature of the culture solution. For example, when using λ phage PR promoter or PL promoter which is activated by temperature increase, the expression of the recombinant protein during growth can be suppressed when the temperature of the culture solution during the growth is in the range of 20 to 37°C, and the expression of the recombinant protein can then be induced by increasing the temperature of the culture solution to 38 to 44°C. In order to lessen the effect of a heat shock protein during the process, a pH of the culture solution during the growth can be adjusted to 6.5 to 7.5, as described in JP H6-292563 A, and the pH of the culture solution is varied to 4.5 to 6.5 at the start of the induction of the recombinant protein expression, thereby allowing more stable induction of expression.

There is no particular restriction on the period from the stage of growth of the recombinant cells until the stage of inducing the expression of the recombinant protein, and it can be appropriately set according to the culturing system configuration and the production process design. From the viewpoint of efficient production of the recombinant protein, it is preferred to initiate the induction of the expression of the recombinant protein when the growth of the recombinant cells has reached the metaphase to the anaphase of the logarithmic growth stage.

The growth of the recombinant cells begins from the lag phase or induction phase (the period of delayed increase in the initial cell count), and through the logarithmic growth stage (the period of logarithmic increase to twice the cell count per unit time), reaches the stationary phase (the period where no net change is seen in the number of cells). The metaphase of the logarithmic growth stage is the period in which the cell count is midway between the cell count in the lag phase and the cell count in the stationary phase, and the anaphase of the logarithmic growth stage is the period from the metaphase until the stationary phase. As a specific example of the period for initiating the induction of the recombinant protein expression, for recombinant cells wherein the OD₆₀₀ value at the stationary phase is approximately 150, it is preferably the period in which the OD₆₀₀ value has reached 30 to 110, more preferably the period in which it has reached 40 to 90, and even more preferably the period in which it has reached 50 to 80.

The time for inducing the expression of the recombinant protein may be a time length until the predetermined production volume has been obtained, which will depend on the type of host used and the target protein. Since the production rate varies depending on the culturing conditions such as the temperature of the culture solution, it is not necessary to absolutely specify the time for inducing the expression of the recombinant protein. The time for inducing the expression of the recombinant protein may also be set to match progression to separation and purification of the recombinant protein in the subsequent step. For industrial production, it is preferred to set the time for the induction of the recombinant protein expression so as not to affect the growth of the recombinant cells being carried out in parallel, or transfer of the grown recombinant cells.

### (Preculture step)

The production method for a recombinant protein according to the present embodiment may further include a preculture step. The preculture step is a step of culturing the recombinant cells in a preculture medium before the growth suppression step. Specific aspects of the preculture medium are the same as the aspects of the protein production culture medium described above.

In the production method for a recombinant protein according to the present embodiment, as the preculture medium, it is preferred to use a culture medium higher in nutritional components than the protein production culture medium. This can increase the number of the recombinant cells supplied in the growth suppression step and the production step.

### [Method for increasing production volume of recombinant protein per cell]

The present invention described above can be perceived as a method for increasing a production volume of a recombinant protein per cell. That is, the method for increasing a production volume of a recombinant protein per cell according to an embodiment includes a growth reduction step of reducing cell growth of recombinant cells which express a recombinant protein and a production step of producing the recombinant protein by culturing the recombinant cells in a protein production culture medium in a state of reducing the cell growth, in which the cell growth of the recombinant cells is reduced in the growth reduction step by using recombinant cells having at least one modified morphogenetic regulator as the recombinant cells. Specific and preferred aspects of the method are as described above.

### Examples

The present invention will now be explained in greater detail based on examples. However, the present invention is not limited to the examples described below.

### [Example 1]

### (1) Preparation of recombinant cells (Escherichia coli strain which expresses modified fibroin)

### (Target protein)

Modified fibroin (hereunder also referred to as "PRT966") having the amino acid sequence listed as SEQ ID NO: 2 was designed based on the nucleotide sequence and amino acid sequence for fibroin from *Nephila clavipes* (GenBank Accession No.: P46804.1, GI: 1174415). The amino acid sequence listed as SEQ ID NO: 2 is the amino acid sequence, having substitutions, insertions, and deletions of amino acid residues as compared to *Nephila clavipes* fibroin, for increased productivity, and further having the amino acid sequence listed as SEQ ID NO: 3 (tag sequence and hinge sequence) added to the N-terminus.

Nucleic acid coding for PRT966 was then synthesized. The nucleic acid had an NdeI site added at the 5'-end and an EcoRI site added downstream from the stop codon. The nucleic acid was cloned in a cloning vector (pUC118). The nucleic acid was then subjected to restriction enzyme treatment with NdeI and EcoRI for cleavage, after which it was recombined with a pET-22b(+) vector to obtain a pET-22(+)/PRT966 vector.

### (Integration of modified fibroin expression cassette into Escherichia coli genomic DNA)

Using the *Escherichia coli* BL21(DE3) strain as the host, modified fibroin expression cassettes were integrated into the genomic DNA at three sites by the following procedures (a) to (c), thereby obtaining recombinant cells having three modified fibroin expression cassettes.

### (a)attHK022

A first modified fibroin expression cassette was integrated into the genomic DNA using a mechanism of lysogenization by HK022 phage. This mechanism is a sequence-specific recombination between a specific site in the host genomic DNA (attB site) and a specific site in the phage genome (attP (HK022) site).

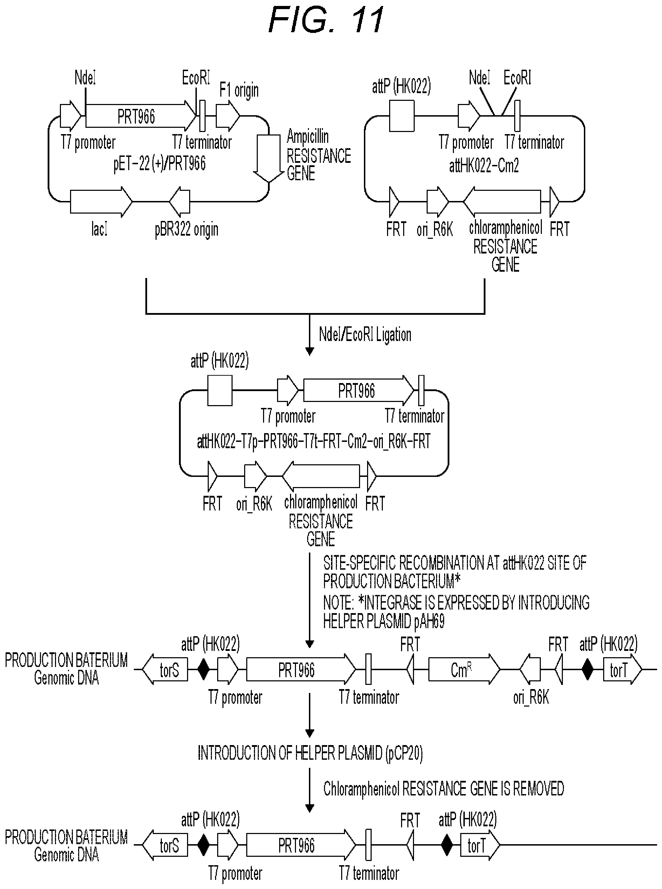

Fig. 11 is a schematic diagram illustrating an outline of a method for integrating the modified fibroin expression cassette into the host genomic DNA using the mechanism of lysogenization by the HK022 phage. First, the pET-22(+)/PRT966 vector was subjected to restriction enzyme treatment with NdeI and EcoRI to cleave the nucleic acid coding for PRT966, which was then recombined with a plasmid vector attHK022-Cm2 having the attP (HK022) site to obtain an attHK022-T7p-PRT966-T7t-FRT-Cm2-ori_R6K-FRT vector. Next, the attHK022-T7p-PRT966-T7t-FRT-Cm2-ori_R6K-FRT vector was introduced into the host, and a modified fibroin (PRT966) expression cassette was integrated into the host genomic DNA by the sequence-specific recombination between the attB site in the host genomic DNA and the attP (HK022) site in the same vector. Note that integrase had been expressed in the host in advance by introducing the helper plasmid pAH69 having the int gene (J. Bact 183: 6384-6393). Then, FLP was expressed by introducing the helper plasmid pCP20 (Proc. Natl. Acad. Sci. USA, 97: 6640-6645), thereby removing the chloramphenicol resistance gene and ori_R6K region flanked by the FRT sequences.

### (b)attϕ80

A second modified fibroin expression cassette was integrated into the host genomic DNA using a mechanism of lysogenization by the ϕ80 phage. This mechanism is a sequence-specific recombination between a specific site in the host genomic DNA (attB site) and a specific site in the phage genome (attP (ϕ80) site).

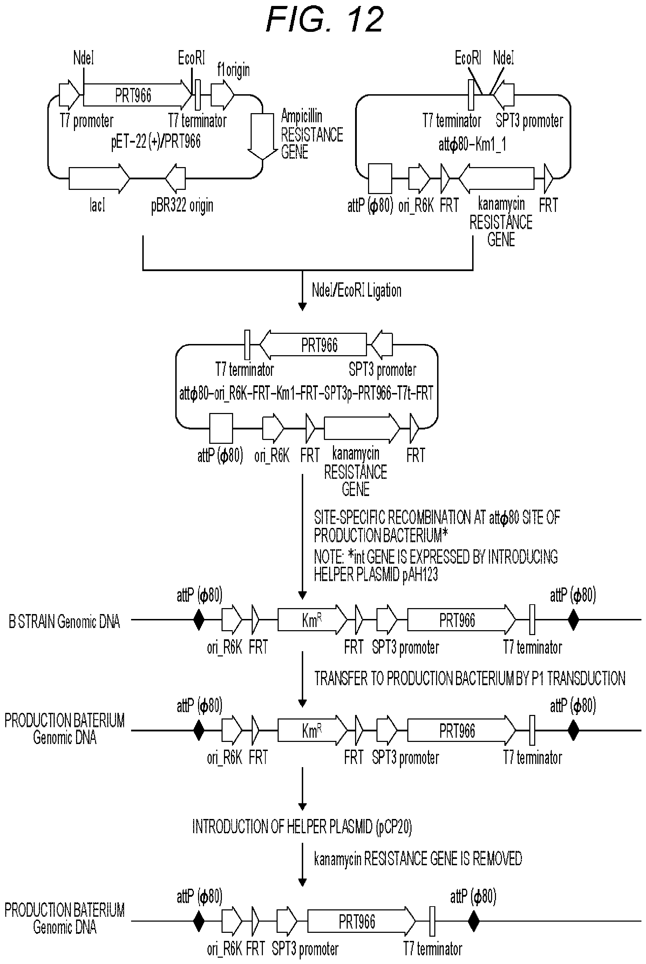

Fig. 12 is a schematic diagram illustrating an outline of a method for integrating the modified fibroin expression cassette into the host genomic DNA using the mechanism of lysogenization by the ϕ80 phage. First, the pET-22(+)/PRT966 vector was subjected to restriction enzyme treatment with NdeI and EcoRI to cleave the nucleic acid coding for PRT966, which was then recombined with a plasmid vector attϕ80-Km1_1 having the attP (ϕ80) site to obtain an attϕ80-ori_R6K-FRT-Km1-FRT-SPT3p-PRT966-T7t-FRT vector. Next, the attϕ80-ori_R6K-FRT-Km1-FRT-SPT3p-PRT966-T7t-FRT vector was introduced into the host which has the first modified fibroin expression cassette integrated therein by the method of (a) above, and a second modified fibroin (PRT966) expression cassette was integrated into the host genomic DNA by the sequence-specific recombination between the attB site in the host genomic DNA and the attP (ϕ80) site in the same vector. Then, FLP was expressed by introducing the helper plasmid pCP20, thereby removing the kanamycin resistance gene flanked by the FRT sequences.

### (c) λ Red_manX

A third modified fibroin expression cassette was integrated into the host genomic DNA using a homologous recombination system of λ phage. In the homologous recombination system, homologous recombination occurs by the products of the exo, bet, and gam genes which are in the Red region in the phage genome.

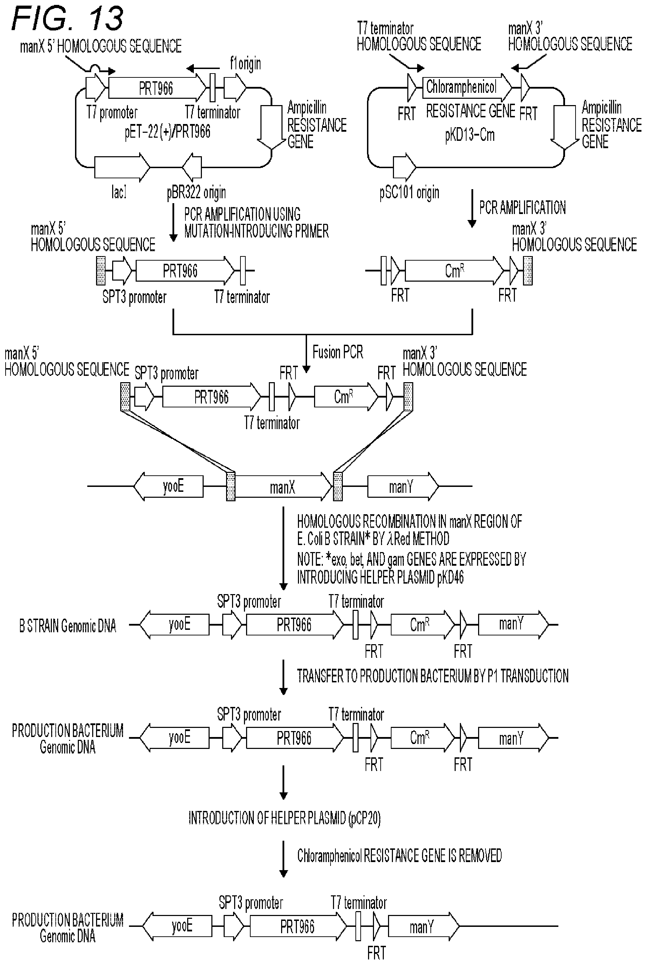

Fig. 13 is a schematic diagram illustrating an outline of a method for integrating the modified fibroin expression cassette into host genomic DNA using the homologous recombination system of the λ phage. First, a modified fibroin expression cassette (including manX 5' homologous sequence-SPT3 promoter-PRT966-T7 terminator in this order) was amplified by a PCR method using the pET-22(+)/PRT966 vector as the template and a primer that introduces a modification into the T7 promoter. Similarly, a chloramphenicol resistance gene expression cassette (including T7 terminator homologous sequence-FRT-chloramphenicol resistance gene-FRT-manX 3' homologous sequence in this order) was amplified by a PCR method using the pKD13-Cm vector as the template. The two PCR products were joined using the In-Fusion (registered trademark) cloning system (manufactured by Takara Bio Inc.). Next, the DNA fragment obtained by the joining was introduced into the host which has the first modified fibroin expression cassette and the second modified fibroin expression cassette integrated therein by the methods of (a) and (b) above, and a third modified fibroin (PRT966) expression cassette was integrated into the host genomic DNA by homologous recombination between the manX 5' homologous sequence in the host genomic DNA and the manX 5' homologous sequence in the DNA fragment and homologous recombination between the manX 3' homologous sequence in the host genomic DNA and the manX 3' homologous sequence in the DNA fragment. Note that the exo, bet, and gam genes had been expressed in the host in advance by introducing the helper plasmid pKD46 having these genes (Proc. Natl. Acad. Sci. USA, 97: 6640-6645). Then, FLP was expressed by introducing the helper plasmid pCP20, thereby removing the chloramphenicol resistance gene flanked by the FRT sequences.

### (2) Introduction of MreB mutation

A region of the MreB gene that codes for the protein (CDS) was obtained by a PCR method and cloned into the pKOV plasmid (J. Bacteriology 179: 6228-6237) using the In-Fusion mix (Takara Bio Inc.). In order to substitute the 53^{rd} amino acid for threonine, the nucleic acid coding for MreB into which a mutation was introduced was amplified by a PCR method using the A2T-F primer (5'-AGCGTAACTGCAGTAGGTCATG-3') and the A2T-R primer (5'-TACTGCAGTTACGCTTTTCGGT-3'), and then the recombinant cells obtained in (1) above was transformed with the nucleic acid after subjecting the nucleic acid to the reaction in the In-Fusion mix. Using the obtained strain, a MreB (A53T) mutant strain was obtained by introducing the MreB-A53T mutation into the genome of the strain by the method described in J. Bacteriology 179: 6228-6237.

### (3) Expression and evaluation of modified fibroin

The recombinant cells obtained by the methods of (1) and (2) above (recombinant cells having three modified fibroin expression cassettes in the genomic DNA and having the MreB (A53T) mutation; hereunder also referred to as "MreB mutant strain") were cultured by the following method, and the expression level of the modified fibroin was analyzed. For comparison, the recombinant cells obtained by the method of (1) above (recombinant cells having three modified fibroin expression cassettes in the genomic DNA and not having a mutation in MreB; hereunder also referred to as "wild-type MreB strain) were also evaluated in the same manner.

The MreB mutant strain and the wild-type MreB strain were each cultured for 15 hours in 2 mL of LB medium. The culture solution was added to 100 mL of preculture medium (seed culture medium of Table 2) to an OD₆₀₀ of 0.005. Flask culturing was conducted to an OD₆₀₀ of 5 (approximately 15 hours) while keeping the culture solution temperature at 30°C, to obtain seed culture solutions.

**[Table 2]**

| Seed culture medium | |
|---|---|
| Reagent | Concentration (g/L) |
| Glucose | 5.0 |
| KH₂PO₄ | 4.0 |
| K₂HPO₄ | 9.3 |
| Yeast Extract | 6.0 |

The seed culture solution was added to a jar fermenter to which 500 mL of a protein production culture medium (production culture medium of Table 3) was added, to an OD₆₀₀ of 0.05. Culturing was conducted while keeping the culture solution temperature at 37°C and controlling the pH to be constant at 6.9. In addition, the dissolved oxygen concentration in the culture solution was maintained at 20% dissolved oxygen saturated concentration.

**[Table 3]**

| Production medium | |
|---|---|
| Reagent | Concentration (g/L) |
| Glucose | 12.0 |
| KH₂PO₄ | 9.0 |
| MgSO₄ ·7H₂O | 2.4 |
| Yeast Extract | 15 |
| FeSO₄·7H₂O | 0.04 |
| MnSO₄·5H₂O | 0.04 |
| CaCl₂·2H₂O | 0.04 |
| ADEKA NOL (ADEKA CORPORATION, LG-295S) | 0.1 (mL/L) |

A feed solution (feed substrate solution of Table 4) was added at a rate of 6 g/hour immediately after the complete consumption of glucose in the protein production culture medium. Culturing was conducted while keeping the culture solution temperature at 37°C and controlling the pH to be constant at 6.9. In addition, the culturing was conducted for 16 hours while maintaining the dissolved oxygen concentration in the culture solution at 20% dissolved oxygen saturated concentration. Thereafter, the expression of the modified fibroin was induced by adding 1 M isopropyl-β-thiogalactopyranoside (IPTG) to the culture solution to a final concentration of 0.1 mM. SDS-PAGE was conducted using the cells prepared from the culture solutions obtained before the addition of IPTG and after the addition of IPTG, and the expression of the target modified fibroin which depended on the addition of IPTG was confirmed by the appearance of a band of the size of the target modified fibroin.

**[Table 4]**

| Feed substrate solution | |
|---|---|
| Reagent | Concentration (g/L) |
| Glucose | 600.0 |
| MgSO₄·7H₂O | 10.0 |

The collected cells were washed with 20 mM Tris-HCl buffer (pH 7.4). The washed cells were suspended in a 20 mM Tris-HCl buffer solution (pH 7.4) containing about 1 mM PMSF, and the cells were disrupted with a high-pressure homogenizer (manufactured by GEA Niro Soavi). The disrupted cells were centrifuged, thus obtaining a precipitate. The obtained precipitate was washed with a 20 mM Tris-HCl buffer solution (pH 7.4) until the precipitate became highly pure. The washed precipitate was suspended in an 8 M guanidine buffer solution (8 M guanidine hydrochloride, 10 mM sodium dihydrogen phosphate, 20 mM NaCl, and 1 mM Tris-HCl, pH 7.0) to a concentration of 100 mg/mL, and the precipitate was dissolved by stirring with a stirrer at 60°C for 30 minutes. After the dissolution, dialysis was conducted with water using a dialysis tube (cellulose tube 36/32 manufactured by Sanko Junyaku Co., Ltd.). A white aggregate protein obtained after the dialysis was collected by centrifugation, moisture was removed with a lyophilizer, and the lyophilized powder was collected, thereby obtaining the modified fibroin (PRT966).

The production volume of the modified fibroin was evaluated by conducting polyacrylamide gel electrophoresis on the obtained lyophilized powder and conducting image analysis using Totallab (Nonlinear Dynamics Ltd.). The production volume of each modified fibroin calculated from the weight of the lyophilized powder (production volume per cell) was calculated as a relative value with the value when the induction time in the wild-type MreB strain was 32 hours considered as 100%.

During the culturing period, a portion of the culture solution was regularly taken, and an average particle size of the recombinant cells was measured with a particle counter/analyzer (CDA-1000, SYSMEX CORPORATION).

### (4) Results

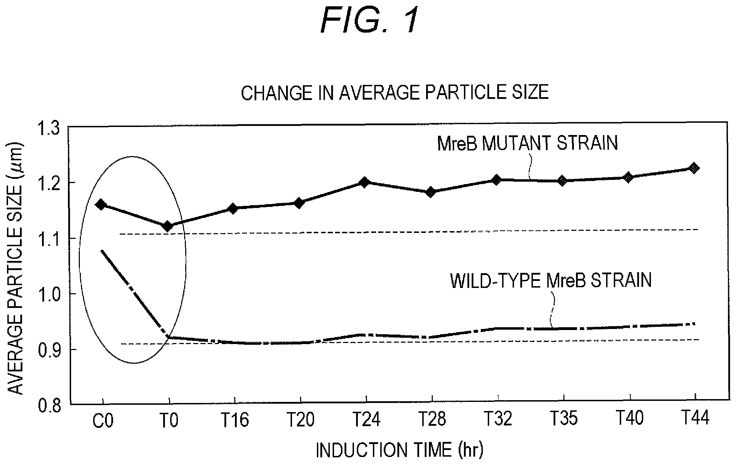

Fig. 1 is a graph obtained by plotting average particle sizes of the MreB mutant strain and the wild-type MreB strain against culturing time. On the horizontal axis, C0 is the point where the seed culturing was switched to the production culturing, and T0 is the point where the production of the recombinant protein started by the expression inducing agent. The MreB mutant strain in the nutrient-rich culture medium during the seed culturing grew by active cell division, and the widths of the cells were larger than those of the wild-type MreB strain. As shown in the period from C0 (point of the transfer to the production culture medium) to T0 (point of the expression induction) in Fig. 1, as the results indicating that, when the strains were then transferred to the production culture medium, the wild-type MreB strain continued the cell division even when the nutritional components in the production culture medium were assimilated, whereas the growth of the MreB mutant strain was slow, the average particle size of the wild-type MreB strain rapidly decreased, whereas the average particle size of the MreB mutant strain scarcely changed, and after the induction of the expression of the modified fibroin, the average particle size of the MreB mutant strain increased by about 30%, compared to the wild-type MreB strain.

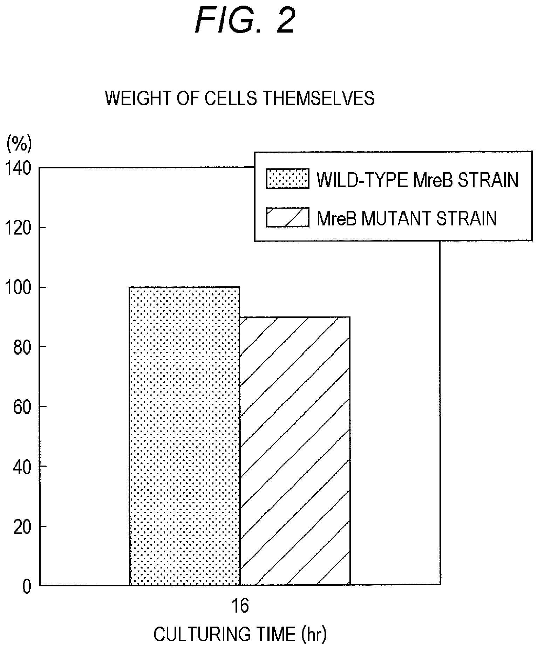

Fig. 2 is a graph showing weights of the cells of the MreB mutant strain and the cells of the wild-type MreB strain themselves (weights obtained by subtracting the weight of the produced recombinant protein from a dry cell weight) 16 hours after the start of the production culturing.

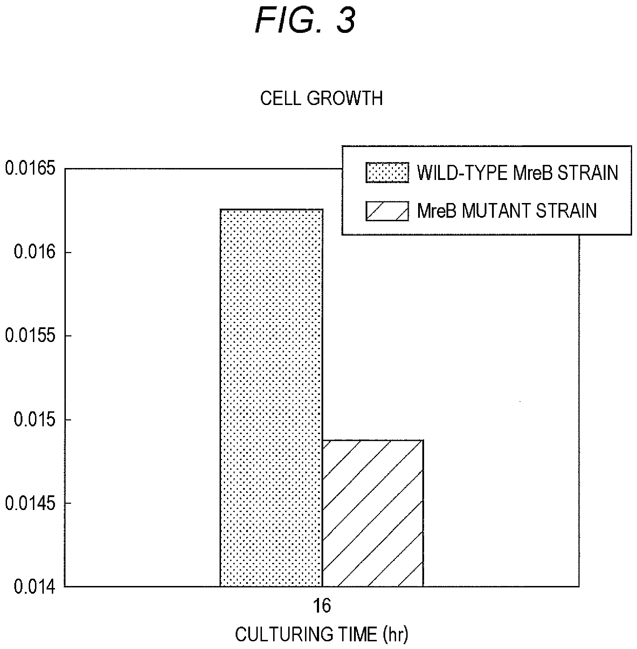

Fig. 3 is a graph showing the growth of the cells of the MreB mutant strain and the cells of the wild-type MreB strain 16 hours after the start of the production culturing. As shown in Figs. 2 and 3, it can be understood that the growth of the MreB mutant strain is reduced compared to that of the wild-type MreB strain.

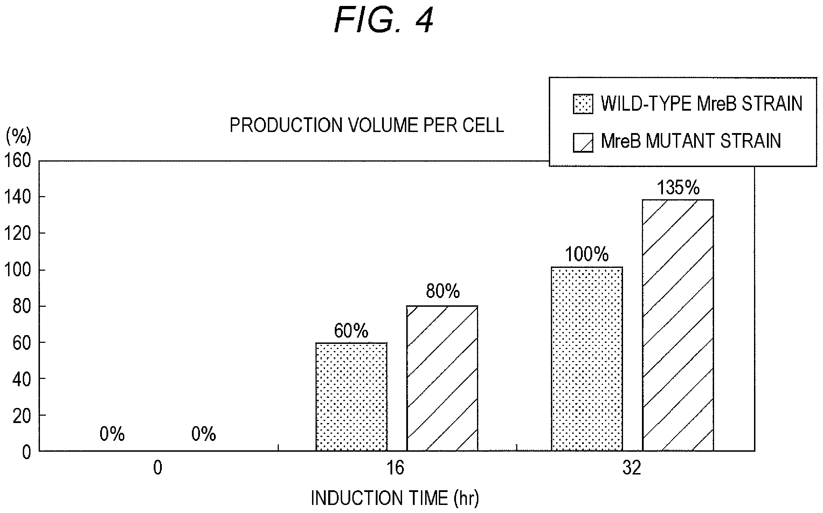

Fig. 4 is a graph showing modified fibroin production volumes (production volumes per cell) in the MreB mutant strain and the wild-type MreB strain. As shown in Fig. 4, the volume of the modified fibroin produced by the MreB mutant strain increased by about 33% 16 hours after the induction of the expression and by about 35% 32 hours after the induction of the expression, compared to the wild-type MreB strain.

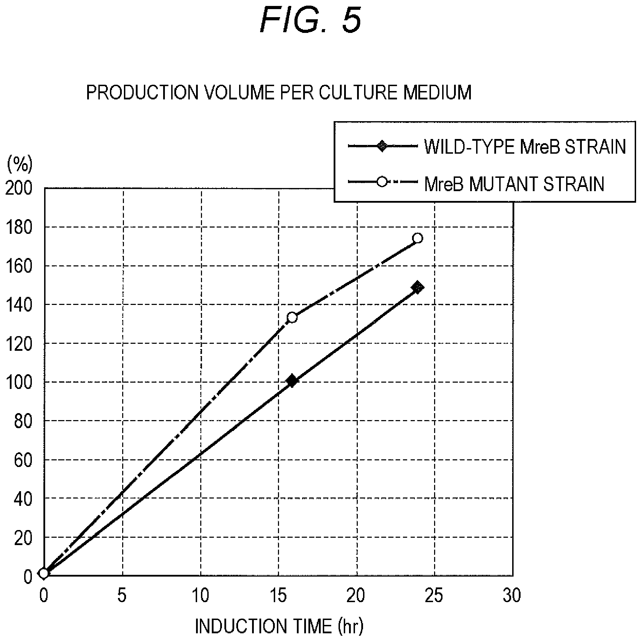

Fig. 5 is a graph showing modified fibroin production volumes (production volumes per culture medium) in the MreB mutant strain and the wild-type MreB strain. As shown in Fig. 5, the volume of the modified fibroin produced by the MreB mutant strain increased by about 33% 16 hours after the induction of the expression, compared to the wild-type MreB strain.

As a result, by using the MreB mutant strain, the yield of the modified fibroin per cell was significantly increased due to the reduction in the cell growth during the modified fibroin production culturing. Furthermore, since the number of cells was smaller compared to the target protein, it was possible to confirm that using the MreB mutant strain in the production of the modified fibroin protein is more advantageous than using the wild-type MreB strain, from the viewpoint of ease of the disrupting operation.

### [Example 2]

### (1) Preparation of Escherichia coli strain which expresses modified fibroin by induction

In the same manner as Example 1, the pET-22(+)/PRT966 vector having modified fibroin PRT966 was obtained. Furthermore, in the same manner as Example 1, using the *Escherichia coli* BL21(DE3) strain as the host, recombinant cells having three modified fibroin expression cassettes were obtained.

### (2) Preparation of Escherichia coli strain which expresses morphogenetic regulator sulA by induction

### (Preparation of vector of morphogenetic regulator expression cassette)

A region of the sulA gene that codes for the protein (CDS) was amplified by PCR using the following primer set sulA_F and sulA_R. In addition, a plasmid vector attP21-KmR2 having an attP (P21) site was linearized and amplified by PCR using the following primer set RBS-4 and pET-MCS_F. The two amplified fragments were joined using NEBuilder HiFi DNA Assembly Master Mix (New England Biolabs Japan Inc.) according to the attached manual to obtain an attP21-T7p-sulA-T7t-FRT-KmR2-ori_R6K-FRT vector.
sulA_F: 5'-TTTAAGAAGGAGATATACATATGTACACTTCAGGCTATGCAC-3' (SEQ ID NO: 8)
sulA_R: 5'-TGTCGACGGAGCTCGAATTCTTAATGATACAAATTAGAGTGAATTTTTAGCCCGG-3' (SEQ ID NO: 9)
RBS-4: 5'-ATGTATATCTCCTTCTTAAAGTTAAACAAA-3' (SEQ ID NO: 10)
pET-MCS_F: 5'-GAATTCGAGCTCCGTCGAC-3' (SEQ ID NO: 11)

To perform the PCR for amplifying sulA, the primers each having the final concentration of 0.2 µM and a BL21(DE3) cell suspension with an OD of about 0.01 were mixed with PrimeSTAR (registered trademark) Max (manufactured by Takara Bio Inc.) according to the attached manual, and a cycle of the temperature of 98°C for 10 seconds, the temperature of 55°C for 5 seconds, and the temperature of 72°C for 30 seconds was repeated thirty times. In the same manner, the PCR for linearizing and amplifying attP21-KmR2 was performed by using the primers each having the final concentration of 0.2 µM and PrimeSTAR (registered trademark) Max (manufactured by Takara Bio Inc.), using 1 ng of the attP21-KmR2 vector as the template, and repeating a cycle of the temperature of 98°C for 10 seconds, the temperature of 55°C for 5 seconds, and the temperature of 72°C for 30 seconds thirty times. The nucleotide sequence of the expression vector thus obtained was confirmed by the Sanger method.

### (Integration of morphogenetic regulator expression cassette into host chromosome)

Next, the recombinant cells obtained in the method of (1) having three modified fibroin expression cassettes in the chromosome thereof were used as host cells, the attP21-T7p-sulA-T7t-FRT-KmR2-ori_R6K-FRT vector was introduced into the host, and a sulA expression cassette was integrated into the host chromosome by sequence-specific recombination between the attB site in the host chromosome and the attP (P21) site in the same vector. Note that integrase had been expressed in the host in advance by introducing the helper plasmid pAH121 having the int gene (J. Bact 183: 6384-6393).

### (3) Expression and evaluation of modified fibroin

The recombinant cells obtained by the methods of (1) and (2) above (recombinant cells having three modified fibroin expression cassettes in the chromosome and having the sulA expression cassette; hereunder also referred to as "sulA induction expression strain") were cultured using the same method as in Example 1, and the expression level of the modified fibroin was analyzed. For comparison, the recombinant cells obtained by the method of (1) above (recombinant cells having three modified fibroin expression cassettes in the chromosome and not having the sulA expression cassette; hereunder also referred to as "control strain") were also evaluated in the same manner.

During the culturing period, a portion of the culture solution was regularly taken, and a particle concentration (cell concentration) of the recombinant cells was measured with a particle counter/analyzer (CDA-1000,

### SYSMEX CORPORATION).

### (4) Results

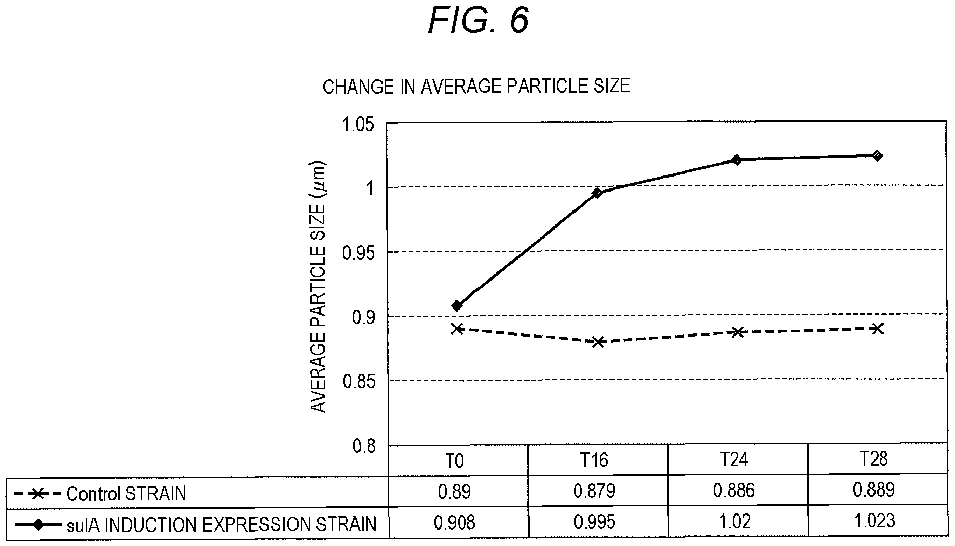

Fig. 6 is a graph obtained by plotting average particle sizes of the sulA induction expression strain and the control strain against culturing time. On the horizontal axis, T0 is the point where the production of the recombinant protein (modified fibroin) started by the expression inducing agent, and the number after T represents the amount of time elapsed since the induction of the expression. After the induction of the modified fibroin expression, the average particle size of the sulA induction expression strain increased, whereas the average particle size of the control strain scarcely changed.

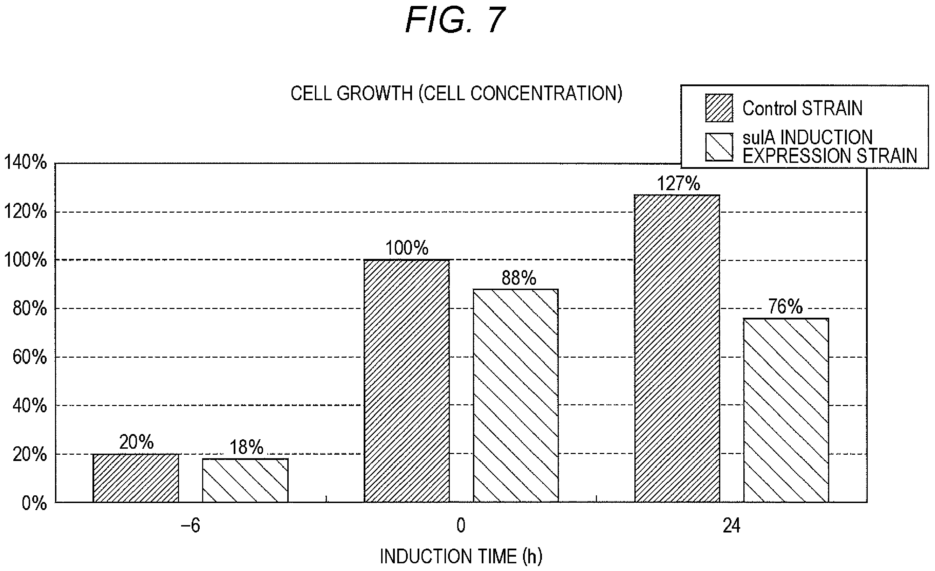

Fig. 7 is a graph showing the change in the relative values of cell concentration (cell count) in the control strain and the sulA induction expression strain when the control strain at the point where the expression of the modified fibroin is induced (T0) is considered as the reference (100%). As shown in Fig. 7, the cell concentration (cell count) was increased to 127% in the control strain 24 hours after the induction of the modified fibroin expression, whereas the cell concentration (cell count) in the sulA induction expression strain decreased.

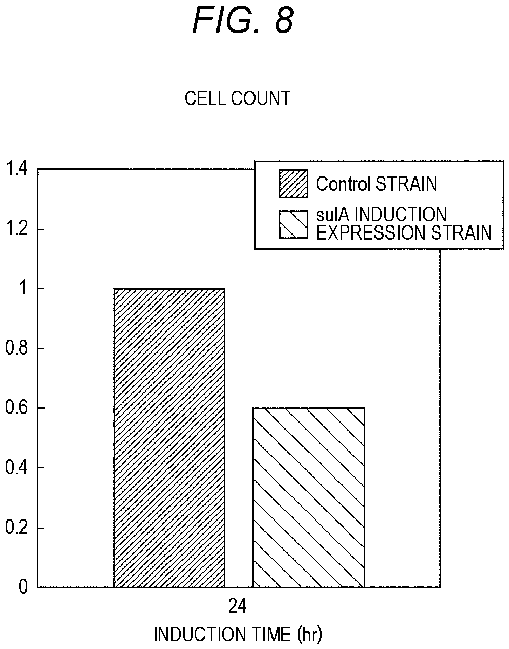

Fig. 8 is a graph showing the relative value of cell concentration (cell count) in the sulA induction expression strain with respect to the control strain 24 hours after the induction of the modified fibroin expression (100%).

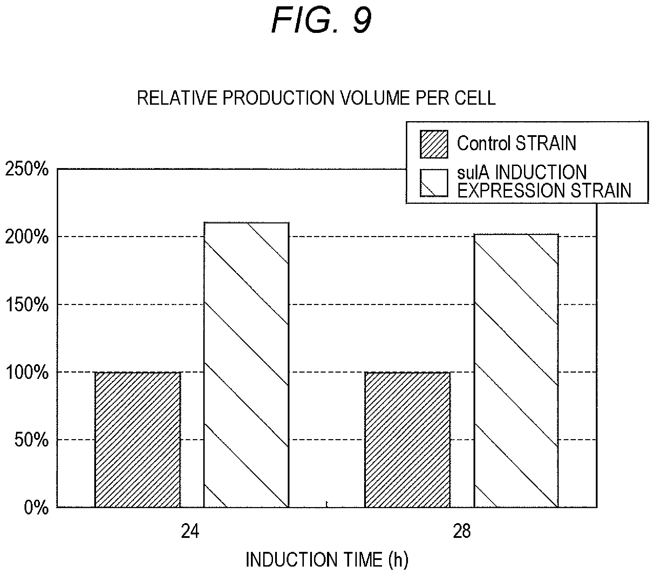

Fig. 9 is a graph showing the relative values of the volume of the modified fibroin produced by the sulA induction expression strain per cell when the control strain is considered as the reference (100%). As shown in Fig. 9, the volume of the modified fibroin produced by the sulA induction expression strain per cell increased by about 200% or more 24 hours and 28 hours after the induction of the expression, compared to the control strain.

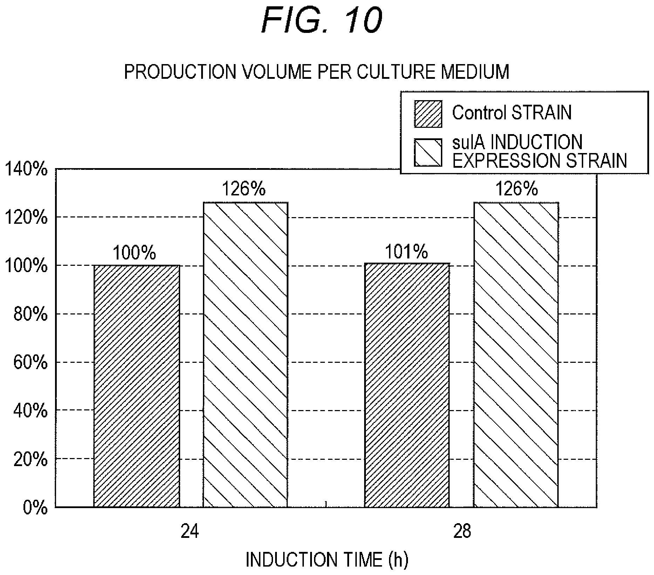

Fig. 10 is a graph showing the relative values of the volume of the modified fibroin produced by the sulA induction expression strain per medium when the control strain is considered as the reference (100%). As shown in Fig. 10, the volume of the modified fibroin produced by the sulA induction expression strain per medium increased by about 126% 24 hours after the induction of the expression, compared to the control strain.

As a result, by using the sulA induction expression strain, the yield of the modified fibroin per cell was significantly increased due to the reduction in the cell growth during the modified fibroin production culturing. Furthermore, since the number of cells was smaller compared to the target protein, it was possible to confirm that using the sulA induction expression strain in the production of the modified fibroin protein is more advantageous than using the general recombinant cells which express the modified fibroin (control strain), from the viewpoint of ease of the disrupting operation.

## Claims

1. A production method for a recombinant protein, the production method comprising:
a growth reduction step of reducing cell growth of recombinant cells which express a recombinant protein; and
a production step of producing the recombinant protein by culturing the recombinant cells in a protein production culture medium in a state of reducing the cell growth,
wherein the cell growth of the recombinant cells is reduced in the growth reduction step by using recombinant cells having at least one modified morphogenetic regulator as the recombinant cells.

2. The production method according to claim 1, wherein the modified morphogenetic regulator is a mutant cytoskeletal protein.

3. The production method according to claim 2, wherein the mutant cytoskeletal protein is mutant MreB.

4. The production method according to claim 3, wherein the mutant morphogenetic regulator has an amino acid sequence having at least 90% sequence identity with MreB.

5. The production method according to claim 3 or 4, wherein the mutant morphogenetic regulator has a mutation at the 53^{rd} amino acid residue of MreB, which is alanine.

6. The production method according to any one of claims 3 to 5, wherein the mutant morphogenetic regulator has a mutation that substitutes the 53^{rd} amino acid residue of MreB, which is alanine, for threonine.

7. The production method according to claim 1, wherein the recombinant cells are cells into which an expression cassette of a protein which controls a function of a cytoskeletal protein is introduced.

8. The production method according to claim 7, wherein the protein which controls the function of the cytoskeletal protein is sulA.

9. The production method according to any one of claims 1 to 8, wherein the protein production culture medium contains a naturally derived component.

10. The production method according to any one of claims 1 to 9, wherein a hydrophobicity of the recombinant protein is -1.0 or higher.

11. The production method according to any one of claims 1 to 10, wherein the recombinant protein is a structural protein.

12. The production method according to any one of claims 1 to 11, wherein the recombinant protein is fibroin.

13. The production method according to any one of claims 1 to 12, wherein the recombinant protein is spider silk fibroin.

14. The production method according to any one of claims 1 to 13, wherein the recombinant cells are bacilli.

15. The production method according to any one of claims 1 to 14, wherein the recombinant cells are microorganisms belonging to the genus *Escherichia.*

16. A method for increasing a production volume of a recombinant protein per cell, the method comprising:
a growth reduction step of reducing cell growth of recombinant cells which express a recombinant protein; and
a production step of producing the recombinant protein by culturing the recombinant cells in a protein production culture medium in a state of reducing the cell growth,
wherein the cell growth of the recombinant cells is reduced in the growth reduction step by using recombinant cells having at least one modified morphogenetic regulator as the recombinant cells.

17. The method according to claim 16, wherein the modified morphogenetic regulator is a mutant cytoskeletal protein.

18. The method according to claim 16, wherein the recombinant cells are cells into which an expression cassette of a protein which controls a function of a cytoskeletal protein is introduced.
